# EUROPEAN PATENT APPLICATION

(11) **EP 0 285 267 A2**
(43) Date of publication of application: **05.10.1988**
(21) Application number: 88301919.2
(22) Date of filing: 04.03.1988
(51) Int. Cl.: C07D 405/04, C07D 211/90, C07D 213/80, C07D 317/62, A61K 31/445, A61K 31/455

(54) **Pyridine derivatives for treatment and prevention of liver damage, and their preparation**

(30) Priority: 05.03.1987 JP 51744/87; 26.10.1987 JP 271432/87
(71) Applicant: YAMANOUCHI PHARMACEUTICAL CO. LTD., Tokyo (JP)
(72) Inventor: Iwamoto, Hidenori, Kitakatsusika-gun Saitama (JP); Satoh, Masato, Itabashi-ku Tokyo (JP); Nagano, Noriaki, Hasuda-shi Saitama (JP); Koide, Tokuo, Itabashi-ku Tokyo (JP); Ikadai, Hiroyuki, Itabashi-ku Tokyo (JP)
(74) Representative: Geering, Keith Edwin

(57) **Abstract**

Pyridine derivatives, their production, and compositions containing them for treatment and prevention of liver damage. These pyridine derivatives have the following structure :

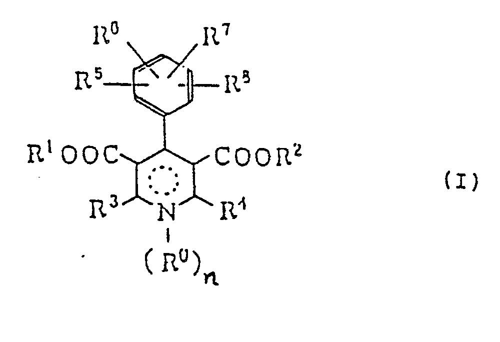

## Description

The present invention relates to pyridine derivatives of formula (I) below, and their salts, which are useful as medical drugs (especially as agents for treatment and prevention of liver damage), and to processes for their production: 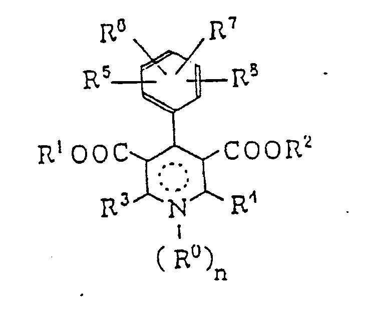 wherein
R¹ and R² are the same or different and selected from lower alkyl groups and groups represented by 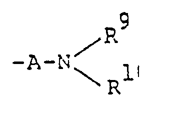 (wherein A is lower alkylene; R' is lower alkyl and R¹⁰ is lower alkyl or aralkyl, or R' and R¹⁰ together form a 5-or 6-membered ring);
R3 and R⁴ are the same or different and selected from lower alkyl groups;
R⁵ and R⁶ are not both hydrogen but otherwise are the same or different and selected from hydrogen and halogen atoms and C₁-C₆ alkoxy, (C₁-C₆ alkoxy)carbonyl and nitro groups;
R⁷ and R¹ together form C₁-C₆ alkylenedioxy (that is, 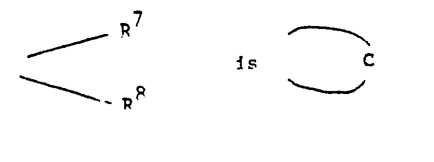 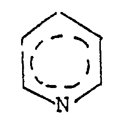 is fully unsaturated or has only a 2,3-position double bond and/or a 5,6-position double bond;
R° is a hydrogen atom lower alkyl group which may be substituted by or a (lower alkoxy)carbonyl or lower alkoxy; and
n is 0 or 1 according to the attached ring structure.

The compounds of the present invention are novel compounds and it is not known that similar pyridine compounds have effect for treatment and prevention of liver damage.

The liver is the largest and metabolically the most complex and active organ of the body. The functions of the liver are numerous and include metabolism, synthesis, detoxification, secretion and storage. All nutrients, toxins, poisons, drugs and viruses pass through the liver and there they are metabolized, detoxified, or converted to other substances by the liver cells (hepatocytes). Therefore, any damage or injury to the hepatocytes causes disturbance of liver. function with severe consequences for normal functioning of the whole body.

For clinical purposes, the liver can be considered in terms of blood supply, parenchymal cells, Kupffer cells and biliary passages. Specific liver diseases tend to affect these four components in a predictable manner, often with characteristic clinical, biochemical and histological consequences.

Symptoms and signs of hepatic dysfunction are numerous and while some may be seen only in chronic liver disease, the others will be observed in both acute and chronic liver disorders.

The liver damage caused by viruses, drugs, toxic chemicals, poisons, alcohol or radiation are characterized by the elevation of the liver enzymes such as glutamic-pyruvic transaminase (GPT), glutamic- oxalic transaminase (GCT), alkaline phosphatase (AP), sorbital dehydrogenase (SDH) and in the level of bilirubin. These tests are most useful in determination of the character and the extent of liver damage and are generally part of routine evaluation of the patient with liver disease. For example, increased activity of serum alkaline phosphatase suggests mechanical obstruction of the extrahepatic biliary tree, an infiltrative lesion of the liver, early manifestation of cirrhosis or drug induced cholestasis. Increase in the serum transaminases (GPT and GOT) and sorbitol dehydrogenase activity is common in all kinds of liver disease and indicates hepatocyte injury. The Merck Manual, 13th Ed., Section 8:836 (1977). Therefore, any decrease in the elevated levels of these enzymes following drug treatment would indicate that the drug is effective in treatment of the liver damage.

Jaundice, one of the most known and visible symptoms of liver disorder, is caused by excess of circulating bilirubin. Any abnormality due to liver disorder in the bilirubin metabolism will cause hyper- bilirubinemia. Thus, any increase in the level of bilirubin is considered a good indicator of liver damage and the determination of serum bilirubin is a necessary part of the routine evaluation of a patient with liver disease. Ibid.

Liver injury is also accompanied by liver cell necrosis affecting particularly parenchymal liver cells. Such necrosis can be determined histologically and is an excellent indicator of the extent of liver injury. Moreover, changes in the magnitude of liver cell necrosis indicate whether drug treatment has been effective and to what extent.

Successful drug treatment of liver damage is, therefore, accompanied by:
1. reducing elevated liver enzymes;
2. decreasing levels of bilirubin; and
3. diminishing the extent of liver cell necrosis.

Because of its importance, liver damage has been widely studied and models simulating hepatic injuries similar or identical to viral or chemical hepatitis have been developed. The models of experimental hepatitis are numerous. Those most resembling actual viral or chemical hepatitis are induced by hepatotox- ins such as D-galactosamine, carbon tetrachloride and bromobenzene.

The administration of D-galactosamine to rats results in a reproducible liver cell injury. Moreover, the biochemical and morphological pattern of resulting liver cell injury is qualitatively related to the dose of D-galactosamine. Since a high dose of D-galactosamine induces reversible structural and functional change in rat livers, it makes an analyzable model for the study of the effect of drugs and pharmaceuticals on liver damage. Carbon tetrachloride is a known model hepatotoxin widely used for studies of acute chemical liver injuries. In the liver, carbon tetrachloride is converted by the liver cells to a metabolite which peroxidizes and alkylates tissue macromolecules. This conversion initiates the stereotypical events of acute lethal liver cell injury. Aside from D-galactosamine, the carbon tetrachloride model is considered most suitable for studies of liver injuries particularly those due to chronic alcoholic ingestion.

The galactosamine and carbon tetrachloride hepatitis models are described, for example, in Amer. J. Path., 79:579,(1975); Virchows Arch. B. Cell. Path., 26:331 (1978), and in Seminars in Liver Disease, 1:143-(1981 ).

The compounds of this invention, namely the formula (I) compounds and salts are novel; we find them to be active in the prevention and treatment of liver damage and, therefore, useful for treatment of viral hepatitis, alcoholic liver disease, drug-, poison-, radiation-and chemotherapy-induced liver disease and toxic hepatitis.

Examples of known compounds are Malotilate, Noscapine, Tritoqualine, etc. The N-containing ring-in the compounds of this invention may be 1,4-dihydropyridine ring, etc. according the position of double bond(s) in the ring represented by 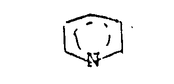 Further, the invention provides a pyridine derivative of following formula (I-B) 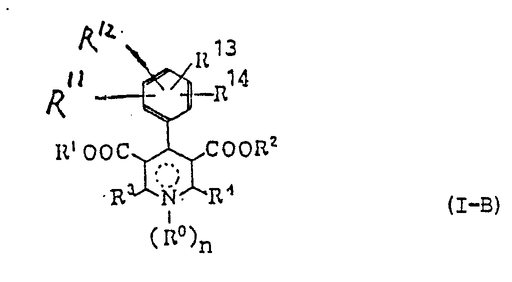 wherein R¹³ and R" are are the same or different and are selected from hydrogen, halogen, lower alkoxy, (lower alkoxy)carbonyl, and nitro; and R¹¹, R¹² are both hydrogen atoms, or together form lower alkylenedioxy (with the proviso that when R" and R¹² are both hydrogens, R¹³ and R¹⁴ are not both hydrogens); and the remaining symbols are as defined in formula I.

The formula I-B compounds cover the formula I compounds and some known compounds also.

In this specification, the term "lower" refers to a straight or branched carbon chain having up to 6 carbon atoms, unless otherwise indicated. Accordingly, concrete examples of the lower alkyl group include methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimsthylpropyl, 1-ethyl-1-methylpropyl, and 1-ethyl-2-methylpropyl groups, etc.

As an "aryl group", there are as concrete examples phenyl and naphthyl groups and the like but particularly preferred is phenyl.

An "aralkyl group" is one obtainable by substituting any hydrogen atom in a "lower alkyl group" with an "aryl group" - e.g. a benzyl, phenethyl or phenylpropyl group, etc.

An alkylene group preferably has 1 to 6 carbon atoms. Specific examples are methylene, ethylene, methylmethylene 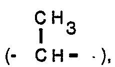 trimethylene, propylene ( 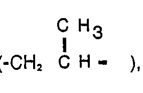 2-propylene 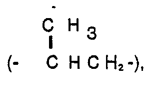 dimethylmethylene 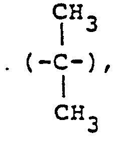 tetramethylene, 1-methyltrimethylene, 2.methyltrimethylene, 3-methyltrimethylene, 1-ethylethylene, 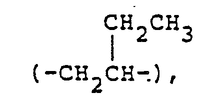 2-ethylethylene 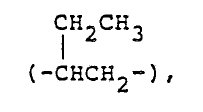 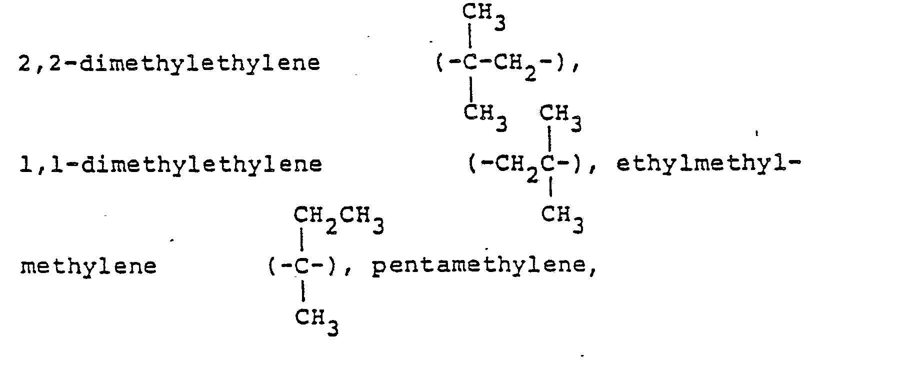 2-methyltetramethylene, 3-methyltetramethylene, 4-methyltetramethylene, 1,1-dimethyltrimethylene, 2,2-dimethyltrimethylene, 3,3-dimethyltrimethylene, 1,3-dimethyltrimethylene, 2,3-dimethyltrimethylene, 1,2-dimethyltrimethylene, 1-ethyltrimethylene. 1,1,2-trimethylethylene, diethylmethylene, hexamethylene, 1-methylpentamethylene, 1,1-dimethyltetramethylene, 2,2-dimethyl tetramethylene, 3,3-dimethyltetramethylene, 4,4-dimethyltetramethylene, 1,1,3-trimethyltrimethylene, 1,1,2-trimethyltrimethylene, 1,1,2,2-tetramethylethylene, 1,1-dimethyl-2-ethylethylene, 1,1-diethyfethylene, heptamethylene, 1-methylhex- amethylene, 1,1-dimethylpentamethylene, 2,2-dimethylpentamethylene, 3,3-dimethylpentamethylene, 4,4-dimethylpentamethylene, 5,5-dimethylpentamethylene, 1,1,4-trimethyltetramethylene, 1,1,2-trimethyltetramethylene, 1,1,3-trimethyltetramethylene, 1,1,2,2-tetramethyltrimethylene, 1,1,3,3-tetramethyltrimethylene, 1,1-dimethyl-2-ethyltrimethylene, 1,1-dimethyl-3-ethyltrimethylene, octamethylene, 1-methyl- heptamethylene, 1,1-dimethylhexamethylene, nonamethylene, 1-methyloctamethylene, 1,1-dimethylhep- tamethylene, decamethylene, 1-methylnonamethylene, 1,1-dimethyloctamethylene, undecamethylene, 1-methyldecamethylene, 1,1-dimethyinonamethylene, dodecamethylene and 1,1-dimethyldecamethylene groups etc.

Eamples of "lower alkylenedioxy" are methylenedioxy, ethylenedioxy, propylenedioxy, etc.

The present invention includes the salts of compounds (I), and examples of such salts include acid addition salts with inorganic acids such as mineral acids (e.g. hydrochloric, hydrobromic, hydroiodic, sulfuric, nitric and phosphoric acids and the like) and with various organic acids such as formic, acetic, oxalic, citric, succinic, fumaric, maleic, malic, tartaric, methanesulfonic and ethanesulfonic acids and the like.

Some compounds according to the invention can evidently have various isomers such as optical isomers, diastereoisomers and geometrical isomers. The present invention includes isolated isomers and any mixtures thereof. That is, the present compounds may have asymmetric carbon at the 4-position of the pyridine ring and/or in certain 4-phenyl substituents (according to whether R⁵, R⁶, R⁷, and R⁸ include an asymmetric carbon.

The present invention also includes processes for producing the compounds represented by the general formula (I). The compounds (I) of the present invention can be prepared by various processes. Representative examples of the processes will be given below.

### General synthesis method:

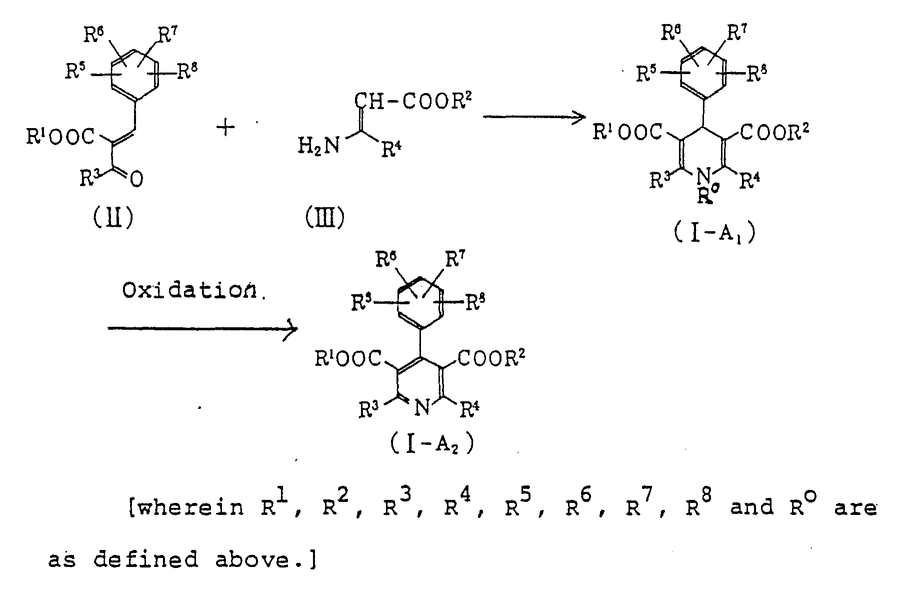

The above formula (I-A,) compounds can be prepared by reacting formula (II) compound (which can be prepared by reacting substituted benzaldehyde with acylacetic derivative) with formula (III) compound. It is generally advantageous to conduct the reaction in solvent which does not take part in the reaction, such as alcohols (for example ethanol, iso-propanol, etc.), dioxane, diemthylformamide, dimethylsulfoxide, acetonitrile, etc. Compounds (II) and (III) can be used in almost equimolar amounts. As the solvents, organic solvents as above or inorganic solvent such as water may be used.

Compounds (II) can be prepared by allowing the substituted benzaldehyde to react with acylacetic acid derivative by azeotropic dehydration; thus obtained compound (II) may be reacted with compound (III) after or without isolation of compound (II). The conversion from compound (I-A,) to compound (I-A2) can be conducted by normal oxidation, such as nitric acid oxidation or nitrous acid oxidation (for example, sodium nitrite-nitric acid, sodium nitrite-sulfuric acid, sodium nitrite-acetic acid). The reaction is preferably conducted at room temperature or under heating.

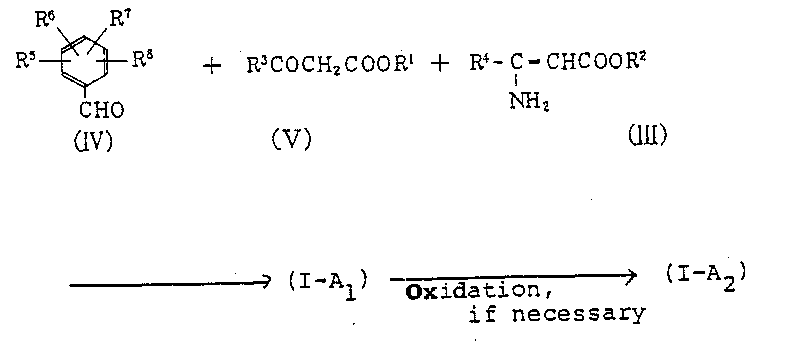

Compounds (I-A,) can also prepared by reacting aldehyde compound (IV), acylacetic derivative (V) and aminocrotonic acid derivative (III), simultaneously.

The reaction can conducted in the absence or presence of any .solvent, e.g. organic solvent such as ethanol, n-propanol, iso-propanol, pyridine, etc. The reaction is preferably conducted at room temperature or with heating or under reflux. The oxidation of compound (I-A, ) to compound (I-Az) can be carried out under similar reaction conditions to (i) above.

Other synthesis method:
Compounds (I-A,) can also prepared by all kinds of reactions for known dihydropyridine compounds. Some example reactions are shown below:

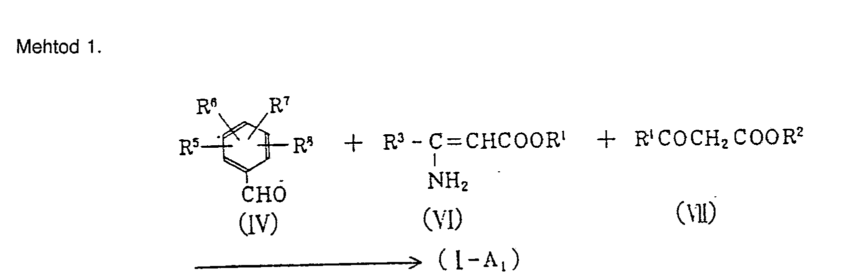

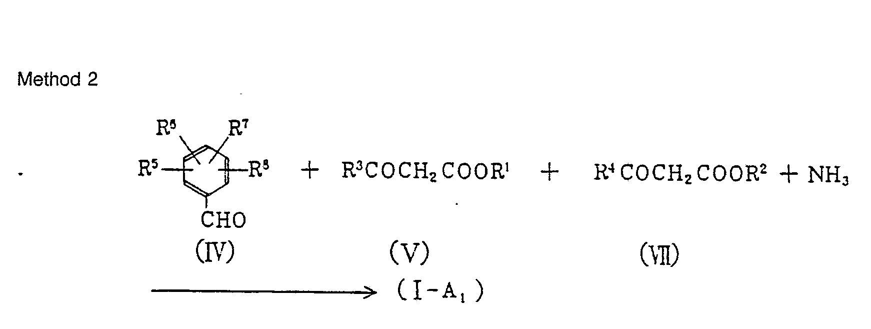

The above two reactions can proceed in the absence of any solvent, but it is generally advantageous to conduct the reaction in a solvent which does not take part in the reaction, for example, organic solvents such as alcohls (for example, methanol, ethanol, etc.), dimethylformamide, dioxane, dimethylsulfoxide, acetonitrile, etc. Compounds (IV), (VI) and (VII) can be used in almost equimolar amounts; and compounds (V), NH₂R°, compounds (IV) and compounds (VII) in the case of Method 2 can be also used in almost equimolar amounts. The reaction is preferably conducted with heating after mixing the above starting materials.

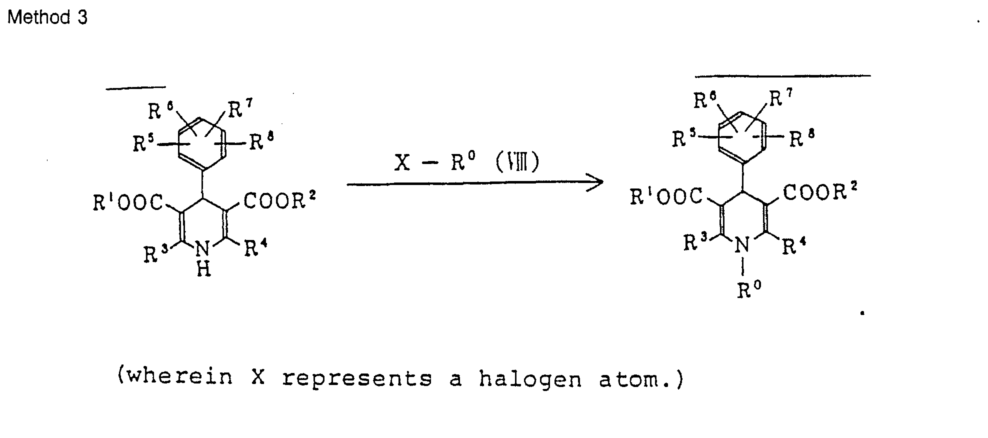

This method is for producing compounds where R° is a substituted alkyl group, and this reaction can be conducted by alkylating compound (I-A,) [namely, dihydropyridine compound wherein R° is hydrogen] with lower-alkylating-agent-compound (VIII) wherein R° is lower alkyl substituted by lower alkoxy or (lower alkoxy)carbonyl. This reaction is conducted by reacting with sodium hydride in a solvent such as tetrahydrofuran, dry dimethylformamide, etc., and then reacting with alkyl halogenide compund (X-R^{a}).

The compounds of this invention can be used as they are, in a free state, or as salts thereof. Isolation and purification can be performed by conventional chemical operations such as extraction, crystallization, recrystallization, various chromatography techniques, etc.

The salts of the compounds of this invention can be prepared using usual salt-forming reactions.

The compounds of this invention are useful for treatment and/or prevention of liver damage, liver disorders, or liver disease. "Treatment" covers any treatment of the. disease in a mammal, particularly human, and includes:
(i) preventing the disease from occuring in a subject which may be predisposed to the disease but has not yet been diagnosed as having it:
(ii) inhibiting the disease, i.e. arresting its development; and
(iii) relieving the disease, i.e. causing regression of the disease.

"Liver damage", "liver disorder", and "liver disease" as used herein mean hepatic and biliary disorders which are caused by viruses, drugs, toxic chemicals, poisons, radiation or alcohol and include all hepatic disease and disorders as described in The Merck Manual, 13th Ed., Section 8, pp:836-886 (1977).

Liver damage may be caused or induced by drugs, toxic chemicals, poisons, radiation, alcohol and viruses. The present compounds can be useful for treatment of such liver disorders as, for example:
1. viral hepatitis type A or type B, mononucleosis, rubella, mumps and coxsackie;
2. alcoholic liver disease, such as fatty liver, steatosis. hepatomegaly, intrahepatic obstructive jaundice, portel hypertension, cirrhosis, fibrosis and acute hepatocellular failure;
3. drug induced liver disease, such as drug induced acote hepatitis. drug induced jaundice or choleostasis;
4. toxic hepatitis caused by such toxic agents as carbon tetrachloride and related hydrocarbons, industrial solvents and phosphorus or other toxic chemicals; or
5. poison hepatitis caused by mushroom poisoning, by poisoning with fungal products known as aflatoxins or by other poisons;
6. radiation and chemotherapy induced liver injury.

The present compounds have an ability to prevent experimentally induced liver damage, as follows;

The experimental liver damage was induced with carbon tetrachloride as the challenging agent. Carbon tetrachloride caused liver damage resembling that of alcohol-induced liver injury.

Male Sprague Dawley rats weighing around 160 grams were used. Carbon tetrachloride was dissolved in olive oil to obtain 20% solution, which was administed in doses of 5ml/kg orally (carbon tetrachloride: 1 ml/ kg). The test compounds were suspended in Cremophor buffer for intraperitoneal administration, and in 0.5% methyl cellulose solution for oral administration; and the test solutions were administered in doses of 5ml/kg respectively. The administration of the test compound was conducted twice, namely, 1 hour before and 3 hours after the administration of carbon tetrachloride. 24 hours after the administration of carbon tetrachloride, blood was collected via the great abdominal vein, and the level of serum GPT was measured. The experimental results show that some compounds of the present invention have activity inhibiting the elevation of GPT due to carbon tetrachloride administration (even an oral administration of 30mg of the compounds). Thus, the compounds of this invention are usuful for agents for treatment and prevention of liver damage.

Table 1 below shows inhibiting effects against the serum GPT elevation due to carbon tetrachloride liver damage, when administering compounds of this invention. Other compounds, of formula I-B, were also found to have activity inhibiting the elevation of GPT due to carbon tetrachloride administration.

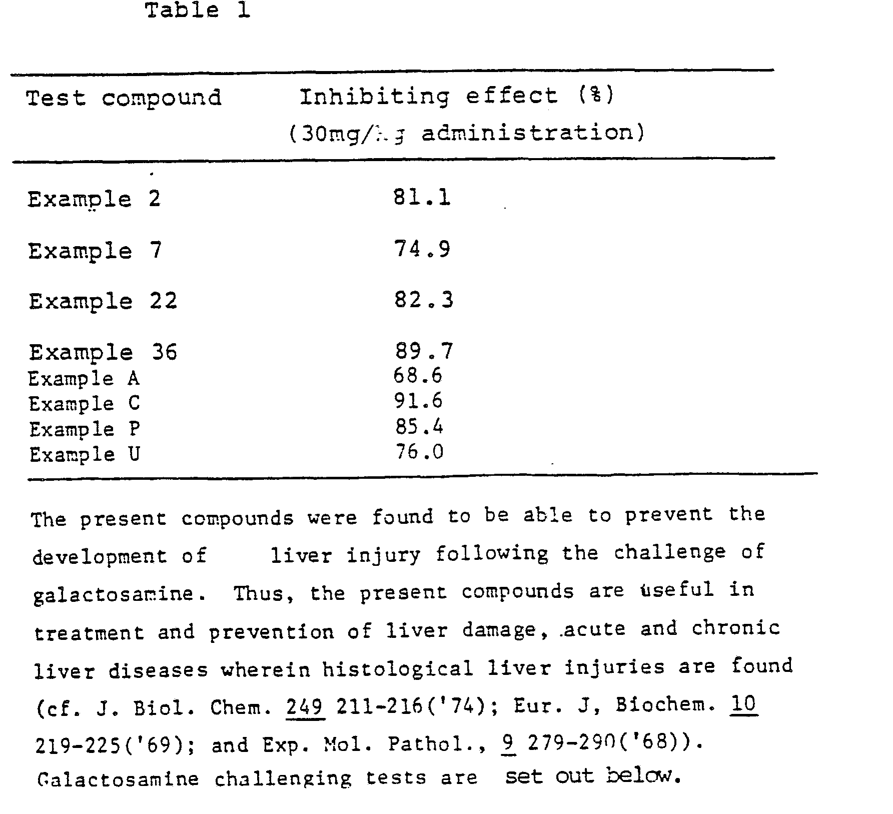

### Prevention of Experimental Liver Damage in Rats:

### D-Galactosamine Challenge

Experimental liver damage was induced with D-galactosamine as the challenging agent. Liver damage caused by D-galactosamine resembled that of acute hepatitis. The galactosamine hepatitis model is described in known prior art. Using this model, we have found that compounds of this application,administered before liver damage occured, effectively prevent the increase in liver enzyme (transaminase) and are thus useful in obviating the liver damage.

### (Experimental procedure)

Animals used: SD male rats (7 week age, about 220 g body weight)

Administration method of the challenge compound:
D-galactosamine was dissolved in a physiological sodium chloride aqueous solution, and this solution was administered intraperitoneally to give galactosamine-induced liver damage. (Administered dose: 425mg/4mllkg) Administration method of the test compound: The test compound was suspended in 0.5 % methyl cellulose solution, and administered in doses of 30 mg/5 mi/kg, orally. The test compound was administered twice (1 hour before the D-galactosamine administration and 3 hours after its administration). 24 hours after the D-galactosamine administration, blood was obtained from the pentobarbital-anesthetized rats,and the level of serum-GPT was determined.

Results are shown in the following Table.

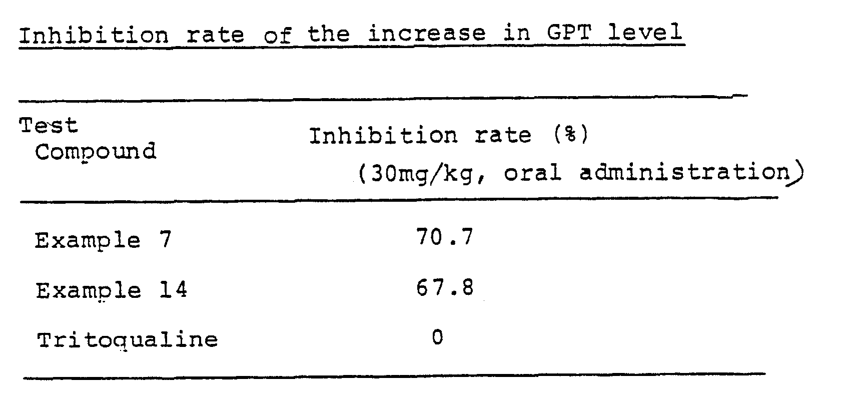

In the above Experiment, Control Compound (Tritoqualine) is difficultly soluble in water, and so no inhibition effect was not recongnized. (The absorbability of Tritoqualine was not good, giving low bioavailability.)

In the following Experiment,an absorbability-increasing agent (Tween 80) was used.

### Animals used: SD male rats (7 week age, about 220 g body weight)

Administration method of the challenge compound:
D-galactosamine was dissolved in a physiological sodium chloride aqueous solution, and this solution was administered intraperitoneally (150mg/5ml/kg, 3 times, 3 hours intervai)to give galactosamine-induced liver damage.
Administration method of the test compound: The test compound was suspended in 0.2% Tween 80, and administered in doses of 10, 30, and 100 mg/5ml/kg , orally. The test compound was administered 1 hour before the first Galactosamine administration. 26 hours after the first Galactosamine administration, blood was obtained from the pentobarbital-anesthetized rats, and the level of serum GPT was determined.
Results are shown in the following Table.

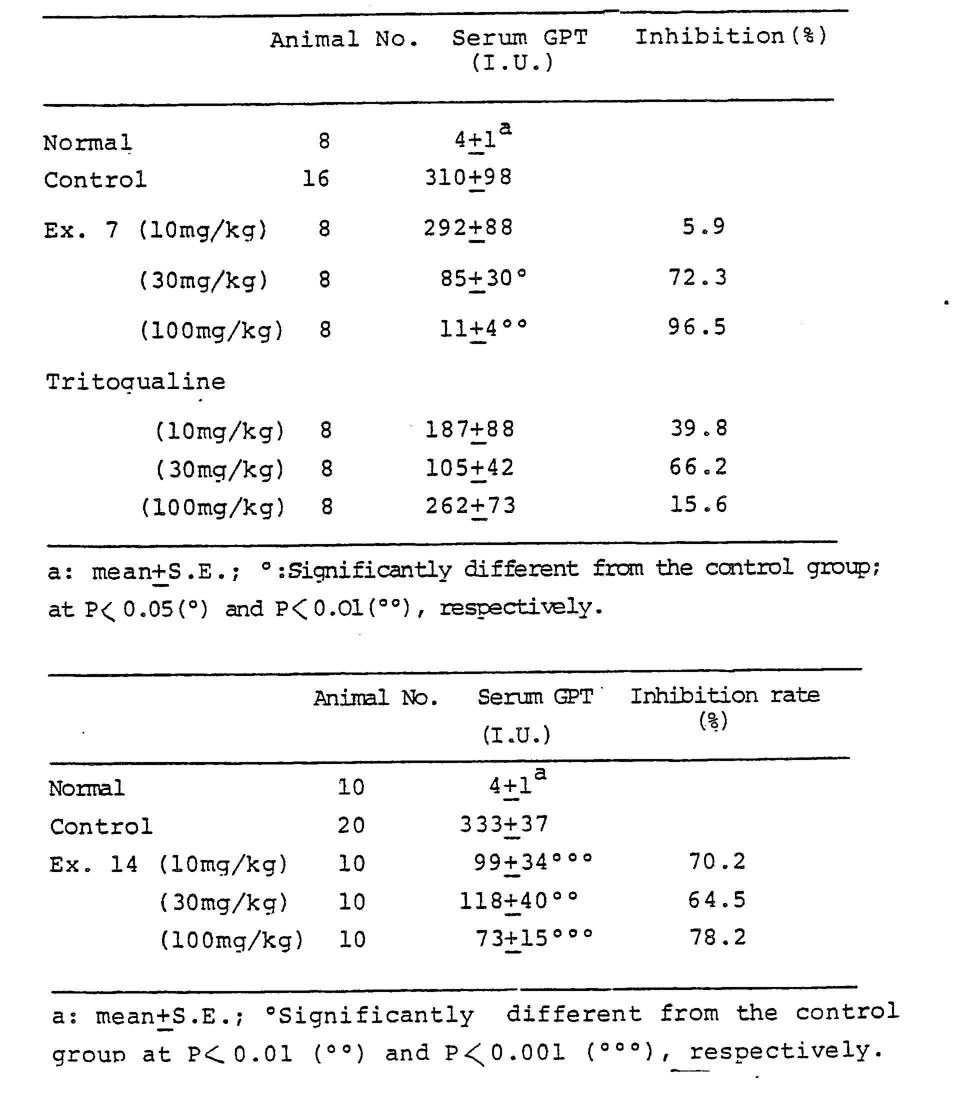

Compositions containing one or more compounds (I) or salts thereof as effective components can be prepared as tablets, powders, granules, granulates, capsules, pills, liquid, injections, suppositories, ointments, etc. using conventional carriers, excipients, etc., and other additives, and can be administered orally (including sublingually) or parenterally.

The carriers and excipients for medical preparations include solid and liquid non-toxic pharmaceutical substances. Examples of such substances are lactose, magnesium stearate, starch, talc, gelatin, agar, pectin, gum arabic, olive oil, sesame oil, cacao butter, ethylene glycol, etc. and other substances ordinarily used.

Clinical dosage of the compounds of the present invention may be appropriately determined depending upon condition, body weight, age, sex, etc. of the patient to be treated, but is generally 10 to 600 mg daily for an adult, administered orally in one dose or several doses.

### Example 1

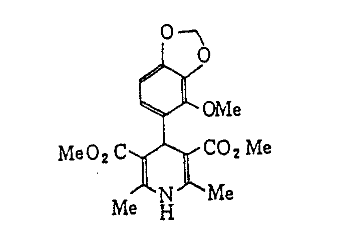

4.50 g of 2-methoxy-3,4-methylenedioxybenzaldehyde was dissolved 45 ml of benzene, and after adding thereto 2.90 g of methyl acetoacetate, 0.15 ml of piperidine and 0.45 ml of acetic acid sucsessively, the mixture was subjected to azeotropic dehydration under reflux. After cooling, benzene was added to the reaction mixture, and then the mixture was washed with water, saturated aqueous sodium hydrogen carbonate, saturated aqueous sodium chloride, successively, and then was dried over anhydrous magnesium sulfate. Magnesium sulfate was removed by filtration, the solvent was distilled away to give crude product of 7.57 g of 2-(2-methoxy-3.4-methylenedioxy)benzylideneacetoacetic acid methyl ester. This crude product of the ester compoumd (7.57 g) was dissolved in 45 ml of iso-propyl alcohol, and after adding thereto 2.88 g of 3-aminocrotonic acid methyl ester, the mixture was refluxed under heating for 20 hours. After cooling, the solvent was distilled away, and the thus obtained crude product was subjected to column chromatography (silica gel: 150 g) and eluted by n-hexane-ethyl acetate (2 : 1 etc. From the n-hexane- ethyl acetate fraction, the product of dimethyl 4-(2-methoxy-3,4-methyienedioxy)phenyl-2,6-dimethyl-1,4-dihydropyridine-3,5-dicarboxylate was obtained.

Melting point: 217 - 220°C (recrystallized from iso-propanol)

Elemental analysis (as C,, H₂1 N07)

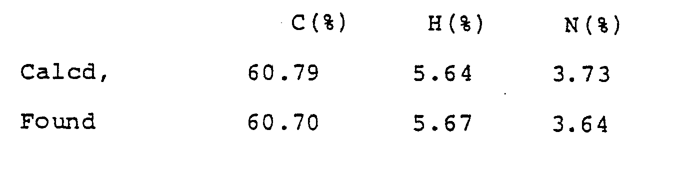

### Example 2

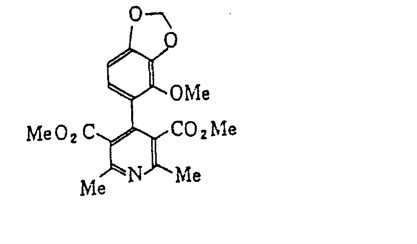

3.00 g of the dihydropyridine compound obtained in Example 1 was dissolved in 80 ml of dioxane-water (1 : 1) by heating, and while stirring at 80°C, 13.2 ml of concentrated nitric acid (61 %; Sp. gr. 1.38) was added to the solution. After 15 minutes. 10 % aqueous sodium hydroxyde (70 ml) was added to the mixture, and the thus obtained mixture was extracted with chloroform (70 ml X 3). The organic layer was washed with water, then with saturated aqueous sodium chloride, and the thus obtained solution was dried over anhydrous magnesium sulfate. After removing magnesium sulfate by filtration, the solvent was distilled away, and the thus obtained crude crystal product was subjected to column chromatography (silica gel: 50 g). From the ether-n-hexane (1 : 1) fraction, dimethyl 4-(2-methoxy-3,4-methylenedioxy)phenyl-2,6-dimethylpyridine-3,5-dicarboxylate was obtained.

Melting point: 120 -122 °C

Elemental analysis (as C,₉ H₁₉ NO₇)

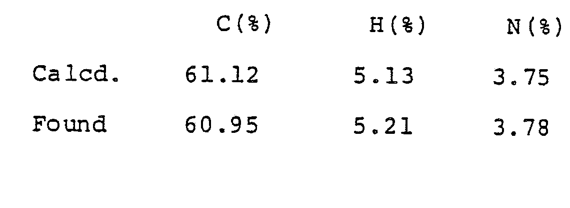

### Example 3

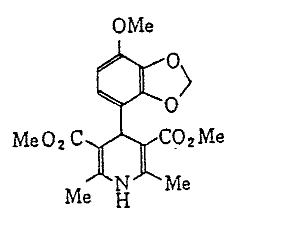

4.50 g of 4-methoxy-2,3-methylenedioxybenzaldehyde was dissolved in 45 ml of benzene, and after adding thereto 2.90 g of methyl acetoacetate, 0.15 ml of piperidine, and 0.45 ml of acetic acid, successively, the mixture was subjected to azeotropic dehydration under reflux for 1.5 hours. After cooling, 45 ml of benzene was added to the reaction solution, and the thus obtained mixture was washed with water, saturated aqueous sodium hydrogen carbonate, and saturated aqueous sodium chloride, successively, and the thus obtained solution was dried over magnesium sulfate. After removing magnesium sulfate by filtration, the solvent was removed by distillation to give crude product (7.81 g ) of 2-(4-methoxy-2,3-methylenedioxy)benzilidene acetoacetic acid methyl ester. 7.81 g of the crude product of the ester compound was dissolved in 45 ml of iso-propyl alcohol, and after adding thereto 2.88 g of 3-aminocrotonic acid methyl ester, the mixture was refluxed under heating for 22 hours. After cooling, the solvent was removed by distillation to give 6.67 g of crude product of dimethyl 4-(4-methoxy-2,3-methylenedioxy)phenyl-2,6-dimethyl-1,4-dihydropyridine-3,5-dicarboxylate.

Melting point: 203 -204 °C (recrystallized from ethyl acetate)

Elemental analysis (as C₁₉ H₂1 N07)

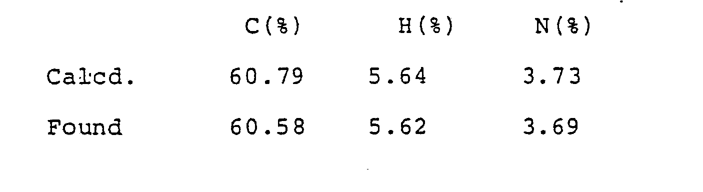

### Example 4

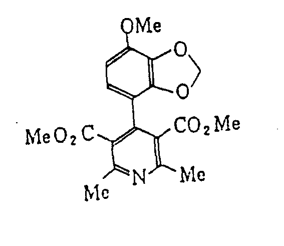

3.00 g of the crude product of the dihydropyridine compound obtained by Example 3 was dissolved in 80 ml of dioxane-water (1 : 1) with heating, and while stirring at 80 °C, conc. nitric acid (61 %; Sp. gr. 1.38) was added dropwise until reddish gas arose. After 15 minutes, 10 % aqueous sodium hydroxide was added to give a mixture of pH 9, and the thus obtained mixture was extracted with chloroform (70 ml X 3) The organic layer was washed with water, and then with saturated aqueous sodium chloride, and the obtained solution was dried over anhydrous magnesium sulfate. After removing magnesium sulfate by filtration, the solvent was removed by distillation, and the thus obtained crude crystal product was subjected to column chromatography (silica gel: 70 g) and eluted by ether-n-hexane (1 : 1). 0.78 g of diemthyl 4-(4-methoxy-2,,B-methyienedioxy)phenyl-2,6-dimethylpyridine-3,5-dicarboxylate was obtained.

Melting point: 128 - 129 °C

Elemental analysis (as C₁₉ H,9 NO,)

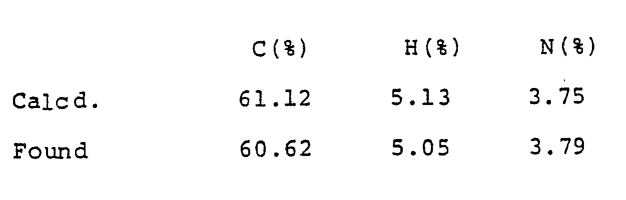

### Example 5

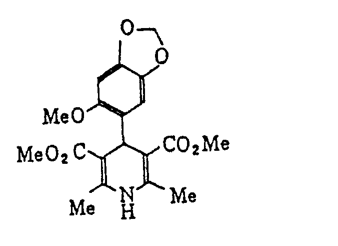

4.50 g of 2-methoxy-4.5-methylenedioxybenzaldehyde was dissolved in 45 ml of benzene, and after adding thereto 2.90 g of methyl acetoacetate, 0.15 ml of piperidine, and 0.45 ml of acetic acid, successively, the mixture was subjected to azeotropic dehydration under heating to reflux for 2 hours. After cooling, 45 ml of benzene was added to thereto, the mixture was washed with water, saturated aqueous sodium hydrogen carbonate, and saturated aqueous sodium chloride, successively, and then was dried over magnesium sulfate. After removing magnesium sulfate by filtration, the solvent was removed by distillation to give7.40 g of crude product of 2-(2-methoxy-4,5-methylenedioxy)benzilideneacetoacetic acid methyl ester. This crude ester compound (7.40 g) was dissolved in 45 ml of iso-propyl alcohol, and after adding thereto 2.88 g of methyl 3-aminocrotonate, the mixture was heated to reflux. After cooling, the solvent was removed by distillation, and the thus obtained crude crustals were subjected to column chromatography (silica gel: 150 g) and eluted with n-hexane-ethyl acetate (2 : 1). 4.37 of dimethyl 4-(2-methoxy-4,5-methylenedioxy)phenyl-2,6-dimethyl-1,4-dihydropyridine-3,5-dicarboxylate was obtained.

Melting point: 208 - 211 °C (recrystallized from ehtyl acetate-n-hexane)

Elemental analysis (as C₁₉ H₂, NO₇)

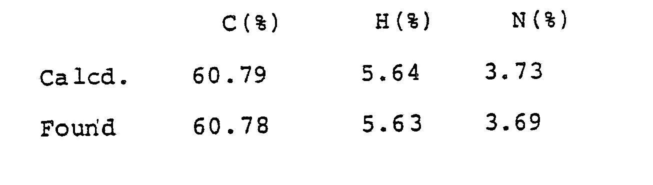

### Example 6

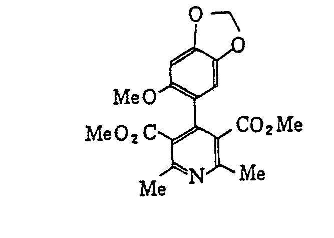

2.50 g of the dihydropyridine compound obtained by Example 5 was dissolved in 90 ml of dioxane-water (2 : 1) by heating, and after adding thereto 11 ml of conc. nitric acid (61 %; Sp. gr. 1.38) at 45 °C, and then adding 1 g of sodium nitrite at 35 °C, the mixture was subjected to reaction. After the reaction was completed, conc. aqueous ammonia was added at 5 - 10° to a pH of 8. The obtained mixture was extracted with chloroform (60 ml x 3), and the organic layer was washed with wather, and then with saturated aqueous sodium chloride the solution thus obtained was dried over magnesium sulfate. After removing magnesium sulfate by filtration, the solvent was removed by distillation, and the thus obtained crude crystal product was subjected to column chromatography (silica gel: 100 g) and eluted with ether-n-hexane (1 : 1). 1.27 g of dimethyl 4-(2-methoxy-4,5-methylenedioxy)phenyl-2,6-dimethylpyridine-3,5-dicarboxylate was obtained.

Melting point: 158 - 160 °C

Elemental analysis (as C,₉ H₁₉ NO,)

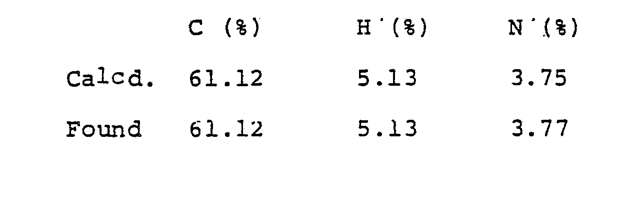

### Example 7

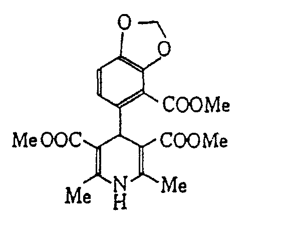

4.7 of 2-methoxycarbonyl-3,4-methylenedioxybenzaldehyde and 2.62 g of methyl acetoacetate was added to 30 ml of benzene, and after adding thereto 0.3 ml of acetic acid and 0.1 ml of piperidine, the mixture was subjected to azeotropic dehydration under heating to reflux. After cooling, 30 ml of benzene was added thereto, and the obtained mixture was washed with water and saturated aqueous sodium hydrogen carbonate, and then saturated aqueous sodium chloride, and further dried over anhydrous magnesium sulfate. The solvent was removed by distillation under reduced pressure. The obtained yellow oily product was dissolved in 50 ml of iso-propanol, and after adding thereto 2.6 g of methyl 3-aminocrotonate, the mixture was heated to reflux for 17 hours. Iso-propanol was removed by distillation under reduced pressure, and the residue was subjected to column chromatography (silica gel column: 500 ml), and eluted by ethyl acetate -n-hexane (1 : 1). 5.5 g of dimethyl 4-(2-methoxycarbonyl-3,4-meth- lenedioxy)phenyl-2,6-dimethyl-1,4-dihydropyridine-3,5-dicarboxylate was obtained.

Melting point: 173 - 174 °C (recrystallized from ehtyl acetate)

Elemental analysis (as C₂₀ H₂, NOₛ)

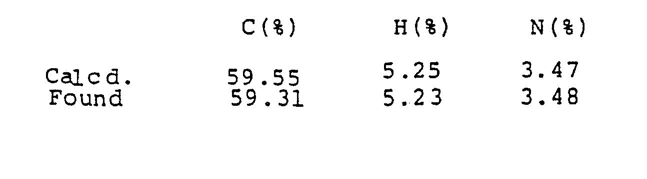

### Example 8

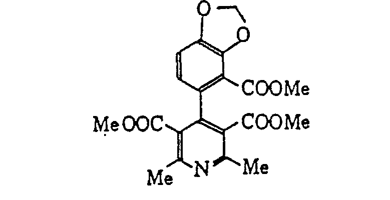

3 g of the dihydropyridine compound obtained by Example 7 was dissolved in 98 ml of dioxane and 196 ml of water, and after adding thereto 24.5 ml of nitric acid (d = 1.38), the mixture was stirred for 1.5 hour at 80 °C. Then, 24.5 ml of nitric acid (d = ₁.₃₈) was added and the mixture was stirred at 80 °C for 0.5 hour. After cooling, the obtained mixture was alkalified with 10 % sodium hydroxide solution, and then extracted with ethyl acetate (200 ml x 2). The extract was washed with saturated sodium chloride solution, and then dried over anhydrous magnesium sulfate. Ethyl acetate was removed by distillation under reduced pressure, and the residue was treated with ether to give 2.5 g of dimethyl 4-(2-methoxycarbonyl-3,4-methlenedioxy)-phenyl-2,6-dimethylpyridine-3,5-dicarboxylate.

Melting point: 142 - 143 °C (recrystallized from ethyl acetate-n-hexane)

Elemental analysis (as C₂₀ H,, NOₛ)

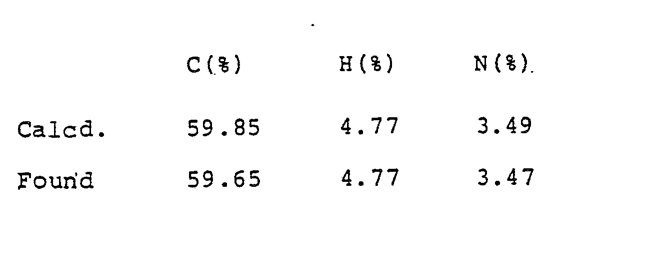

### Example 9

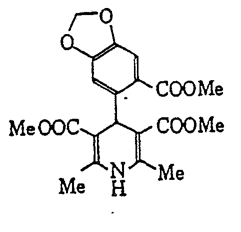

3.30 g of 2-methoxycarbonyl-4,5-methylenedioxybenzaldehyde was dissolved in 30 ml of benzene, and after adding thereto 1.84 g of acetoacetic acid methyl ester, 0.3 ml of acetic acid and 0.1 ml of piperidine, the mixture was subjected to azeotropic dehydration under heating to reflux for 1.5 hour. After cooling, 30 ml of benzene was added thereto, the mixture was washed with water, saturated sodium hydrogen carbonate, and saturated sodium chloride solution, successively. The obtained solution was dried over anhydrous magnesium sulfate, and the solvent was removed by distillation under reduced pressure. The obtained residue was dissolved in 30 ml of iso-propanol, and after adding thereto 1.83 g of methyl 3-aminocrotonate, the mixture was heated to reflux for 20 hours. Iso-propanol was removed by distillation under reduced pressure, and the residue was subjected to silica gel column chromatography (200 ml), using as eluting solution ethyl acetate-n-hexane (1 :2), to give 3.0 g of dimethyl 4-(2-methoxycarbonyl-4,5-meth- lenedioxy)phenyl-2,6-dimethyl-1,4,-dihydropyridine-3,5-dicarboxylate.

Melting point: 167-169 °C

Elemental analysis (as C₂₀ H_{z}, NO₈)

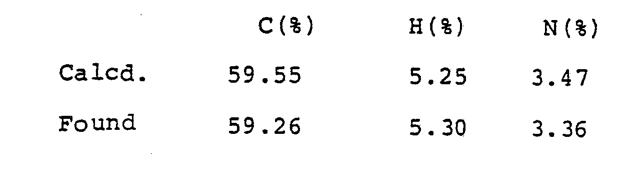

### Example 10

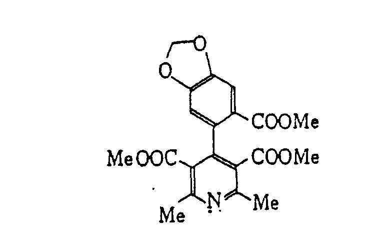

2.1 g of the dihydropyridine compound obtained by Example 9 was dissolved in 70 ml of dioxane and 140 ml of water, and the solution was heated to 80 °C. 34.3 ml of nitric acid (d-1.38) was added thereto portion by portion. After cooling, the mixture was alkalified with 10 % sodium hydroxide solution, and the thus obtained solution was extracted with ethyl acetate (70 ml x 2). The extract was washed with water, saturated sodium chloride solution, successively, and dried over anhydrous magnesium sulfate. Ethyl acetate was removed by distillation under reduced pressure, and the residue was recrystallized from ethyl acetate-n-hexane mixture to give 1.2 g of dimethyl 4-(2-methoxycarbonyl-4,5-methlenedioxy)phenyl-2,6-dimethylpyridine-3,5-dicarboxylate.

Melting point: 123 - 124 °C

Elemental analysis (as C₂0 H₁₉ NO.)

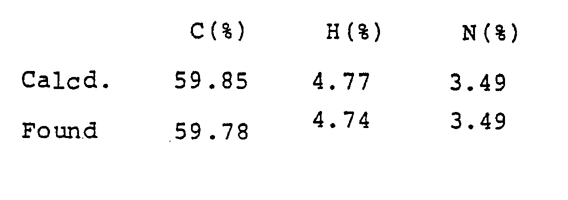

### Example 11

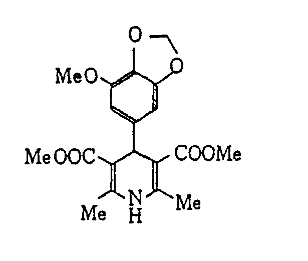

3.1 g of 3-methoxy-4,5-methlenedioxybenzaldehyde and 2.0 g of methyl acetoacetate was dissolved in 30 ml of benzene and after adding thereto 0.3 ml of acetic acid and 0.1 ml of piperidine, the mixture was subjected to azeotropic dehydration under heating to reflux for 3 hours. After cooling, 30 ml of benzene was added to the mixture; and the thus obtained mixture was washed with water, saturated sodium carbonate solution, and saturated sodium chloride solution, successively, and was dried over anhydrous magnesium sulfate. Benzene was removed by distillation under reduced pressure, and the obtained crystals were dissolved in 30 ml of iso-propanol, and after adding thereto 1.98 g of methyl 3-aminocrotonate, the mixture was heated to reflux for 17 hours. Iso-propanol was. removed by distillation, and the residue was recrystallized from a ethyl acetate-n-hexane mixture to give 4.3 g of dimethyl 4-(3-methoxy-4,5-meth- lenedioxy)phenyl-2,6-dimethyl-1,4-dihydropyridine-3,5-dicarboxylate.

Melting point: 180 - 181 °C

Elemental analysis (as C,₉ H₂₁ NO₈)

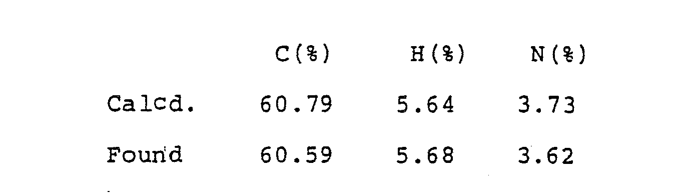

### Example 12

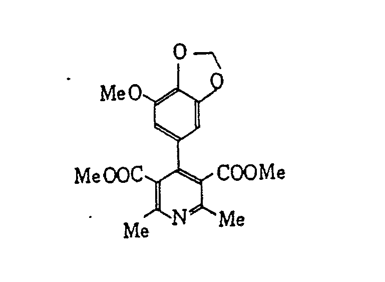

3g of the dihydropyridine compound obtained by Example 11 was dissolved in 39 ml of dioxane and 39 ml of water, and the solution was heated to 70 °C. 13.2 ml of nitric acid was added to the mixture portionwise and the mixture was stirred for 15 minutes. After cooling 10 % sodium hydroxide was added to the mixture to alkalify it, and the alkalified mixture was extracted with chloroform (70 ml x 3). The extract was washed with water and then with saturated sodium chloride solution and further dried over anhydrous magnesium sulfate. Chloroform was removed by distillation under reduced pressure, and the residue was recrystallized from ethyl acetate-n-hezane mixture to give 2.35 g of crystals of dimethyl 4-(3-methoxy-4,5-methylenedioxy)phenyl-2,6-dimethylpyridine-3,5-dicarboxylate.

Melting point: 126 - 127 °C

Elemental analysis (as C,₉ H₁₉ N07)

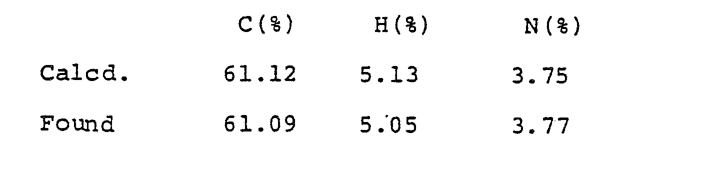

### Example 13

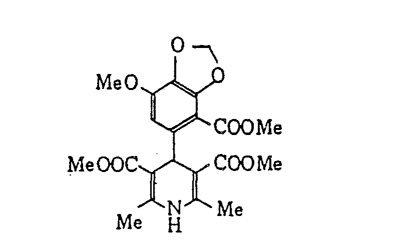

3.12 g of 2-methoxycarbonyl-3,4-methylenedioxy-5-methoxybenzaldehyde and 1.67 g of methyl acetoacetate were dissolved in 50 ml of benzene, and after adding thereto 0.3 ml of acetic acid and 0.1 ml of piperidine, the mixture was heated to reflux for 2.5 hours for azeotropic dehydration. After cooling, 50 ml of benzene was added to the mixture, and the mixture was washed with water, saturated sodium hydrogen carbonate and saturated aqueous sodium chloride, and then dried over anhydrous magnesium sulfate. The solvent was removed by distillation and the residue obtained was dissolved in 50 ml of iso-propyl alcohol, and after adding thereto 1.66 g of methyl 3-aminocrotonate, the mixture was heated to reflux for 18 hours. After cooling, isopropanol was removed by distillation under reduced pressure, and the thus obtained residue was treated with ethyl acetate-n-hexane mixture (1 : 1) to give 2.8 g of dimethyl 4-(2-methoxycarbonyl-3,4-methylenedioxy-5-methoxy)-2,6-dimethyl-1,4-dihydropryidine-3,5-dicarboxylate.

Melting point 214 - 215°C (ethyl acetate-n-hexane)

Elemental analysis (as C₂, H₂₃ NO,)

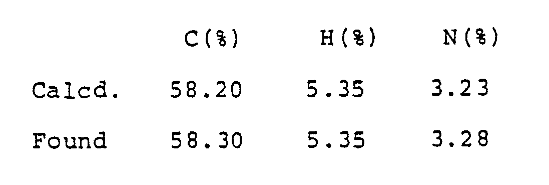

### Example 14

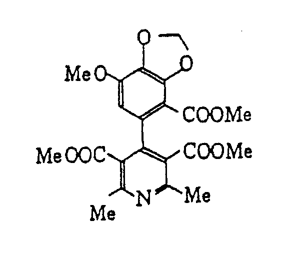

2.08 g of the dihydropyridine compound obtained by Example 13 was suspended in 20 ml of dioxane and 20 ml of water, and after adding thereto 373 mg of sodium nitrite while cooling at 10 °C, nitric acid (d=1.38) (4 ml) was added thereto dropwise gradually. After the mixture reached room temperature, the mixture was stirred for 15 minutes, and alkalified with 10 % sodium hydroxide. The alkalified mixture was extracted with 70 ml of ethyl acetate twice, and the extract solution was washed with water and saturated sodium chloride solution, and then dried over anhydrous magnesium sulfate. The solvent was removed by distillation under reduced pressure, and the residue was recrystallized from ethyl acetate to give 1.72 g of dimethyl 4-(2-methoxycarbonyl-3,4-methylenedioxy-5-methoxy-2.6-dimethylpyridine-3,5-dicarboxylate.

Melting point: 179 - 180 °C

Elemental analysis (as C_{Z}, H₂, NO,)

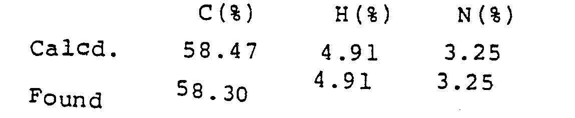

Pharmaceutical Formulation (capsules) [Ingredients]
The Example 14 compound 30 mg
Crystalline cellulose 40 mg
Crystalline lactose 129 mg
Magnesium sulfate 1 mg

1,000 times the above amounts of these ingredients are mixed in ordinary manner, and introduced into gelatin capsules. capsule, 200 mg)

### Example 15

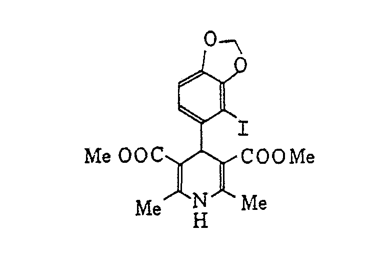

2.5 g of 2-iodopiperonal and 1.05 g of acetoacetic acid methyl ester was dissolved in 20 ml of benzene, and after adding thereto 0.16 ml of acetic acid and 0.05 ml of piperidine, the mixture was subjected to azeotropic dehydration under heating to reflux for 2 hours. After cooling, 20 ml of benzene was added to the reaction mixture, and the mixture was washed with water, saturated aqueous sodium hydrogen carbonate, and saturated sodium chloride, and then dried over anhydrous magnesium sulfate. After removing the solvent by distillation, the obtained residue was dissolved in 16.2 ml of iso-propanol. After adding to the mixture 1.04 g of methyl aminocrotonate, the mixture was heated to reflux for 20 hours. After cooling the mixture with ice-water, the precipitated crude crystals were collected by filtration. The obtained crude crystals were dissolved in 30 ml of ethyl acetate with heating, and after cooling the solution with ice-water, the precipitated crystals were collected by filtration to give 1.22 g of dimethyl 2,6-dimethyl-4-(2-iodo-3,4-methylenedioxy)phenyl-1,4-dihydropyridine-3,5-dicarboxylate.

Melting point: 235 - 236 °C (ethyl acetate)

Elemental analysis (as C₁₈ HIs N, O₆ J)

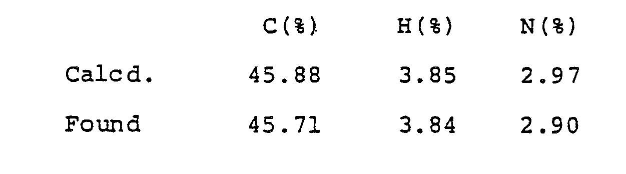

### Example 16

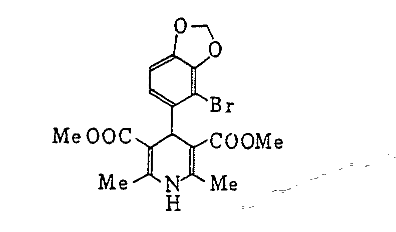

In similar manner to Example 15, 2.46 g of dimethyl 2,6-dimethyl-4-(2-bromo-3,4-methylenedioxyphenyl)-1,4-dihydropyridine-3,5-dicarboxylate was obtained by using 2.60 g of 2-bromopiperonal as a starting material.

Melting point: 235 - 236 °C (ethyl ether) ..

Elemental analysis (as C₁₈ H,, NO₆ Br)

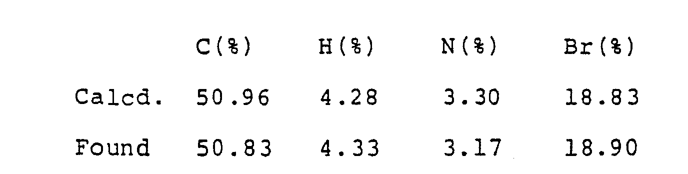

### Example 17

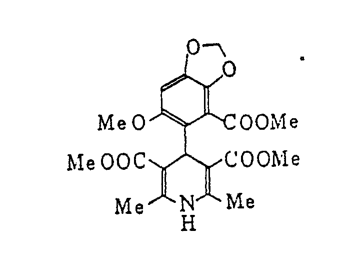

6 g of 2-methoxycarbonyl-3,40methylenedioxy-6-methoxy-benzaldehyde was dissolved in a mixture of 30 ml of benzene and 30 ml of toluene, and after adding thereto 3.2 g of methyl acetoacetate, 0.25 ml of piperidine and 0.8 ml of acetic acid, the mixture was subjected to dehydration reaction under heating to reflux for 3 hours. The solvent was removed by distillation under reduced pressure, and the obtained solid material was subjected to silica gel column chromatography (eluting solution: ethyl acetate/n-hexane =1/1) to give 4.4 g of methyl 2-(2-methoxy-6-methoxycarbonyl-4,5-methylenedioxy)benzilideneacetoacetate (Z'E=1/3). (In this case, if necessary, the Z compound and the E compound can be isolated separately.) 1.0 g of this compound was dissolved in 15 ml of iso-propanol, and after adding thereto 345 mg of methyl 3-aminocrotonate, the mixture was heated to reflux for 3 hours. After cooling, isopropyl alcohol was removed by by distillation under reduced pressure, and the residue was subjected to silica gel column chromatography (eluting solution: ethyl acetate/n-hexane = 1/1). The starting material (290 mg) was recovered; and 425 mg of dimethyl 4-(2-methoxycarbonyl-3,4-methylenedioxy-6-methoxy)phenyl-2,6-dimethyl-1,4-dihydropyridine-3,5-dicarboxylate was obtained.

Melting point: 200.5 -201.5 °C (ethyl aceate - n-hexane)

Elemental analysis (as C₂₁ H₂₃ NO,)

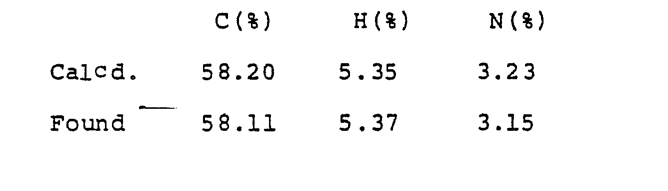

### Example 18

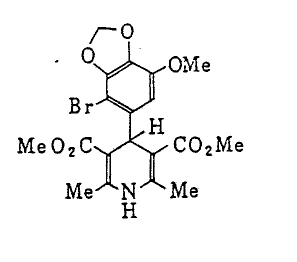

2.2 g of 2-bromo-5-methoxy-3,4-methylenedioxybenzaldehyde (8.5mmol) was dissolved in in 100 ml of benzene, and after adding thereto 1.0 ml of acetoacetic acid methyl ester (9.3 mmol), 0.4 ml of acetic acid and 0.3 ml of piperidine, the mixture was heated to reflux for 2 hours for azeotropic dehydration. After cooling, 100 ml of benzne was added to the mixture, and after washing the mixture with water, saturated sodium hydrogen carbonate solution and brine successively, the mixture was dried over anhydrous magnesium sulfate, and filtered. Benzene was removed by distillation to give 3.2 g of 3.2 g of yellow crystals. The crystals were dissolved in 40 ml of iso-propanol, and after adding thereto 1.0 g (8.7 mmol)of methyl 3-aminocrotonate, the mixture was heated to reflux for 24 hours. After cooling, the precipitated crystals was collected by filtration, and after washing the crystals obtained with iso-propanol, the crystals were subjected to silica gel column chromatography and eluted by ethyl acetate. After recrystallication from n-hexane-methylene chloride, 2.0 g of colorless crystals of dimethyl 4-(2-bromo-5-methoxy-3,4-meth- lenedioxy)phenyl-2,6-dimethyl-1,4-dihydropyridine-3,5-dicarboxylate were obtained (yield: 52 %).

Melting point: 230 - 232 °C

Elemental analysis (as C,, H₂₀ NO₇ Br)

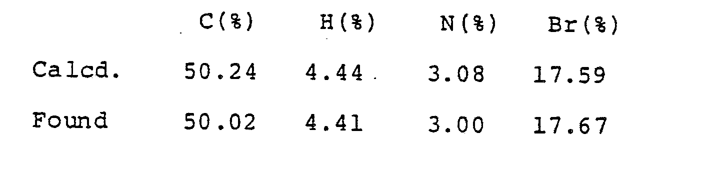

### Example 19

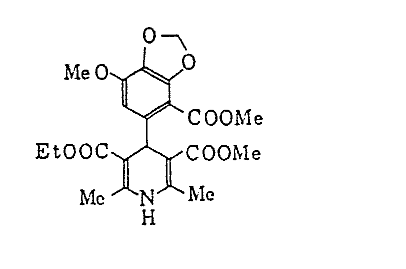

2.35 g of 2-methoxycarbonyl-3,4-methylenedioxy-5-methoxybenzaldehyde was dissolved in 60 ml of benzene, and after adding thereto 1.42 g of ethyl acetoacetate, 0.2 ml of piperidine and 0.6 ml of acetic acid, the mixture was heated to'reflux for 3 hours to be subjected to azeotropic dehydration. After cooling, the mixture was washed with water, saturated sodium hydrogen carbonate solution and saturated sodium chloride solution successively, and dried over magnesium sulfate (anhyrous). The solvent was removed by distillation under reduced pressure, and the residue was dissolved in 80 ml of iso-propyl alcohol. After adding to the mixture 1.14 g of 3-aminocrotonic acid methyl eater, the mixture was heated to reflux overnight. The solvent was removed by distillation under reduced pressure, and the residue was recrystallized from ether to give 2.8 g of 4-(2-methoxycarbonyl-3,4-methylenedioxy-5-methoxy)phenyl-2,6-dimethyl-1,4-dihydropyridine-3.5-dicarboxylate.

### Example 20

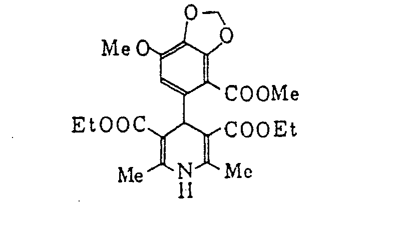

2.38 g of 2-methoxycarbonyl-3,4-methylenedioxy-5-methoxybenzaldehyde was dissolved in 50 ml of benzene, and after adding thereto 1.43 g of ethyl acetoacetate, 0.2 ml of piperidine and 0.6 ml of acetic acid, the mixture was heated to reflux for azeotropc dehydration for 3 hours. After cooling, the mixture was washed with water, saturated sodium hydrogen carbonate solution and saturated sodium chloride solution successively, and dried over anhydrous magnesium sulfate. The solvent was removed by distillation under reduced pressure, and the residue was dissolved in 50 ml of iso-propyl alcohol, and after adding thereto 1.42 g of 3-aminocrotonic acid ethyl ester, the mixture was heated to reflux for 24 hours. The solvent was removed by distillation under reduced pressure, and the residue was treated with ether to give 2.1 g of diethyl 4-(2-methoxycarbonyl-3,4-methylenedioxy-5-methoxy)phenyl-2,6-dimethyl-1,4-dihydropyridine-3,5-dicarboxylate.

Melting point : 201 - 202 °C (ethyl acetate-n-hexane)

Elemental analysis (as C₂₃ H27 NOg)

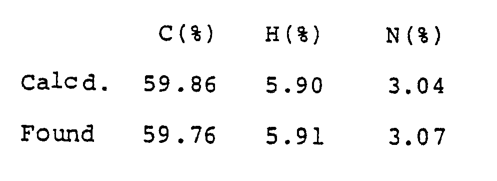

In similar manner to Example 20, the follwing compounds were obtained

### Example 21

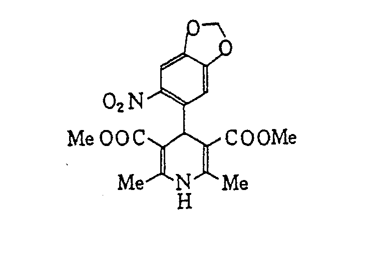

Dimethyl 4-(3,4-methlenedioxy-6-nitro)phenyl-2,6-dimethyl-1,4-dihydropyridine-3.5-dicarboxyfate Melting point: 222 - 223 °C (ethyl acetate)

Elemental analysis (as C₁₈ H₁₈ N₂ 0,)

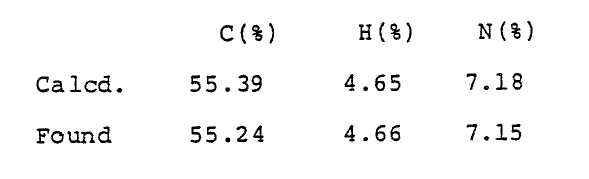

Starting material: 6-nitropiperonal

### Example 22

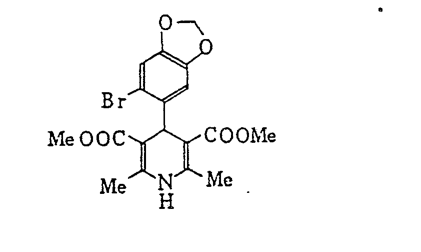

Dimethyl 4-(3,4-methylenedioxy-6-bromo)phenyl-2,6-dimethyl-1,4-dihydropyridine-3,5-dicarboxylate Melting point: above 250 °C (ethyl acetate)

Elemental analysis (as C₁₈H₁₈NO₆Br)

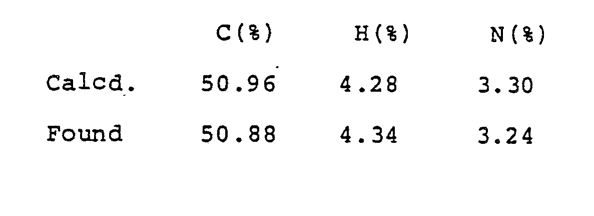

### Example 23

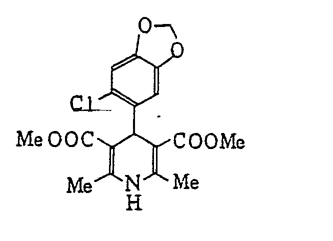

Dimethyl 4-(3,4-methylpnedioxy-6-chloro)phenyl-2,6-dimethyl-1,4-dihydropyridine-3,5-dicarboxylate Melting point above 250 °C (ethyl acetate)

Elemental analysis (as C₁₈H₁₈NO₆Cl)

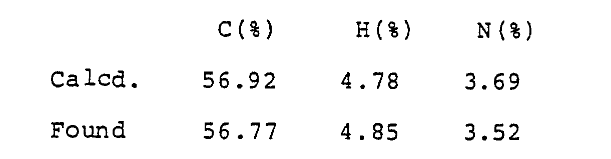

Starting material: 6-chloropiperonal

### Example 24

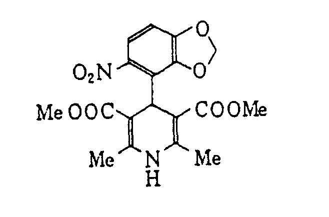

Dimethyl 4-(2,3-methylenedioxy-6-nitro)phenyl-2,6-dimethyl-1,4-dihydropyridine-3,5-dicarboxylate Melting point: 233 -236 °C (ethyl acetate)

Elemental analysis (as C₁₈ H,• N₂O₃)

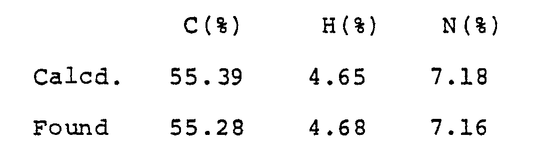

Starting material: 2.3-methylenedioxy-6-nitrobenzaldehyde

### Example 25

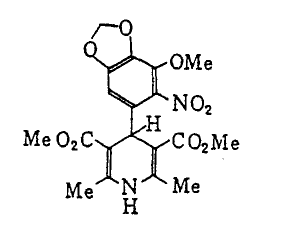

2.5 g of 3-methoxy-4,5-methylenedioxy-2-nitrobenzaldehyde (11.1 mM) was added to 100 ml of benzene, and after adding thereto 1.32 ml (12.2 mM) methyl acetoacetate, 0.4 ml of acetic acid and 0.2 ml of piperidine, the mixture was subjected to azeotropic dehydration by using Dean-Stark system, for 2 hours. After cooling, 200 ml of benzene was added to the mixture, and after washing the mixture with water, 5 % NaHCO₃ and brine successively, the mixture was dried over anhydrous MgOSₐ. Benzene was removed by distillation, and an oily material was obtained (3.5 g). This material was dissolved in 50 ml of 2-propanol and after adding thereto 1.30 g (11.3 mM) of 3-aminocrotonic acid methyl ester, the mixture was heated to reflux for 24 hours. After cooling, the precipitated crystals were collected by filtration, washed with 2-propanol, and recrystallized from hexane-ethanol to give 2.66 g of yellow crystals of dimethyl 4-(3-methoxy-4,5-methylenedioxy-2-nitro)phenyl-2,6-dimethyl-1,4-dihydropyridine-3.5-dicarboxylate. (yield: 59 %)

Melting point: 216 - 218 °C

Elemental analysis (as C₁₉ H₂₀ N₂ O₉)

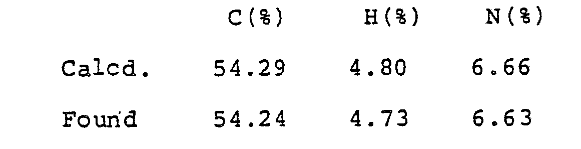

### Example 26

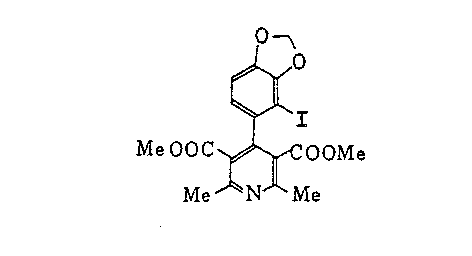

1.5 g of dimethyl 2,6-dimethyl-4-(2-iodo-3,4-methylenedioxyphenyl)-1,4-dihydropyridine-3,5-dicarboxylate was suspended in 10.3 ml of dioxane and 10.3 ml of water, and while cooling at 5 °C, 1.18 ml of conc. nitric acid and then 441 mg of sodium nitrite were added to the mixture. The temperature of the mixture rose to 30 °C, and a transparent solution formed. After stirring the solution at room temperature for 20 minutes, the solution was alkalified with 10 % sodium hydroxide to pH=₉.₂, and was extracted with 50 ml of chloroform twice. The organic layer was washed with water and saturated brine, and dried over anhydrous magnesium sulfate, and after removing the solvent by distillation, crude crystals of the product were obtained. This crystal was dissolved in ethyl acetate-n-hexane mixture (2 : 1) while warming, and after adding thereto 5 ml of n-hexane, the mixture was stirred under ice-cooling to give crystals. The crystals were collected by filtration, and 990 mg of dimethyl 2.6-dimethyl-4-(3-iodo-3,4-methylenedioxyphenyl)-pyridine-3,5-dicarboxylate was obtained.

Melting point: 161.5 - 162 °C (ethyl acetate-n-hexane)

Elemental analysis (as C.ₛ H₁₆ NO₆I)

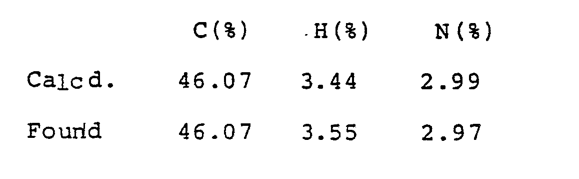

### Example 27

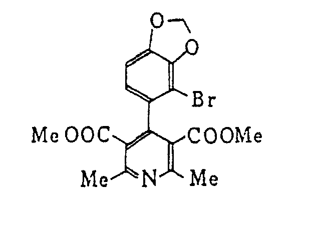

In similar manner to Example 26, 1.2 g of dimethyl 2.6-dimethyl-4-(2-bromo-3,4-methylenedioxyphenyl)-1.4-dihydropyridine-3,5-dicarboxylate (1.5 g) was used as starting material, and 1.2 g of dimethyl 2,6-dimethyl-4-(2-bromo-3,4-methylenedioxyphenyl)pyridine-3,5-dicarboxylate was obtained.

Melting point: 143 - 144 °C (ethyl ether-n-hexane)

Elemental analysis (as C,, H₁₆ NO₆ Br)

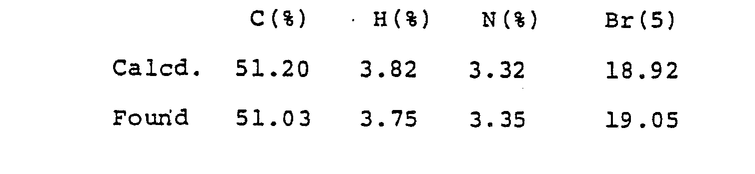

### Example 28

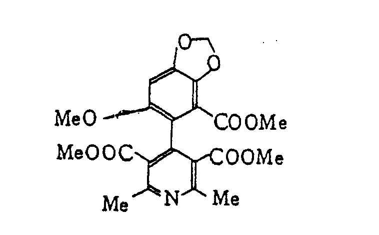

659 mg of dimethyl 4-(2-methoxycarbonyl-3,4-methylenedioxy-8-methoxy)phenyl-2,6-dimethyl-1,4-dihydropyridine-3,5-carboxylate was suspended in 5 ml of dioxane and 5 ml of water, and the mixture was cooled to 12 °C. After adding conc. nitric acid (d = 1.38) (560 µl) and then 220 mg of sodium nitrite, the temperature of the mixture rose to 240 °C. The transparent solution thus obtained was stirred for 20 minutes at room temperature and the solution was alkalified to pH-9.0 with 10 % sodium hydroxide. The alkalified solution was extracted with 30 ml of chloroform and then with 20 ml, and the extract solution was washed with water and saturated sodium chloride, and dried over anhydrous magnesium sulfate. The solvent was removed by distillation, and the caramel material thus obtained was treated with ethyl ether and n-hexane to give 583 mg of dimethyl 4-(2-methoxycarbonyl-3,4-methylenedioxy-6-methoxyphenyl)-2,6-dimethylpyridine-3,5-dicarboxylate.

Melting point: 138 - 139 °C

Elemental analysis (as C₂₁ H₂1 NO₉)

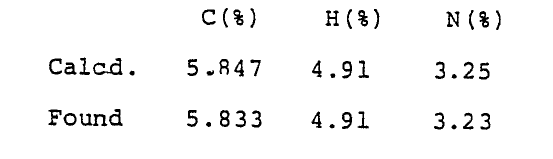

In similar manner to Example 21, the following compounds were obtained.

### Example 29

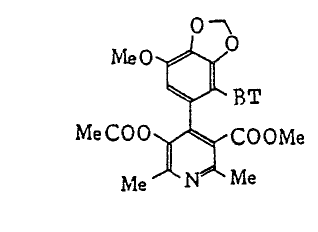

Dimethyl 4-(2-bromo-5-methoxy-3,4-methylenedioxy)phenyl-2,6-dimethylpyridine-3,5-carboxylate Melting point: 167 - 172 °C (ethyl acetate-n-hexane)

Elemental analysis (as C₁₉ H,g NO₇Br)

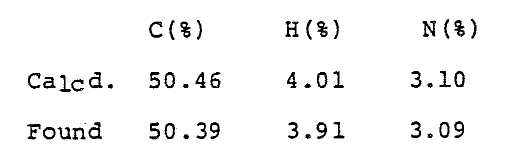

Starting material: Dimethyl 4-(2-bromo-5-methoxy-3.4-methylenedioxy)phenyl-2,6-dimethyl-1,4-dihydropyridine-3,5-dicarboxylate

### Example 30

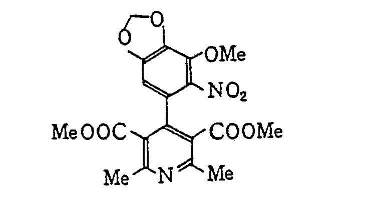

Dimethyl 4-(3-methoxy-4,5-methylenedioxy-2-nitro)phenyl-2,6-pyridine-3,5-dicarboxy(ate Melting point: 173 - 175 °C (ethyl acetate-n-hexane)

Elemental analysis (as C₁₉ H₁₈ N₂O₉)

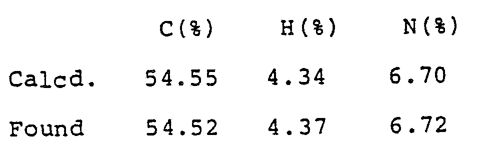

Starting material: Dimethyl 4-(3-methoxy-4,5-methylenedioxy-2-nitro)phenyl-2,6-dimethyl-1,4-dihydropyridine-3,5-dicarboxylate

### Example 31

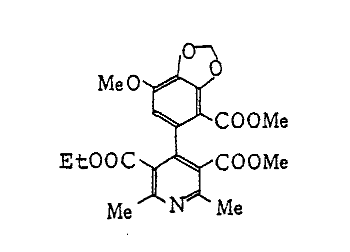

1.0 g of ethyl methyl 4-(2-methoxycarbonyl -3,4-methylenedioxy-5-methoxy)phenyl-2,6-dimethyl-1,4-dihydropyridine-3,5-dicarboxylate was suspended in a mixture of 10 ml of dioxane and 10 ml of water, and after adding thereto 1.65 ml of nitric acid (d = 1.38) and 1.82 mg of sodium nitrite, the mixture was stirred for 30 minutes at room temperature. The mixture was alkalified with 10 % sodium hydroxide solution, and extraced with chloroform. The extract was washed with water and saturated sodium chloride and dried, and the solvent was removed by distillation under reduced pressure. The residue was treated with ether to give 920 mg of ethyl methyl 4-(2-methoxycarbonyl-3,4-methylenedioxy-5-methoxy)phenyl-2,6-dimethylpyridine-3,5-dicarboxylate.

Melting point: 145 -146 °C (ethyl acetate-n-hexane)

Elemental analysis (as C₂₂ H₂₃ NO₉)

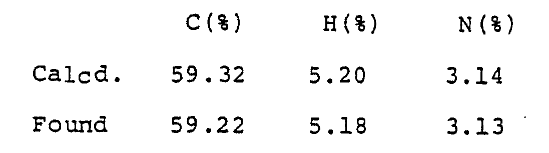

In similar manner to Example 31, the following compounds were obtained.

### Example 32

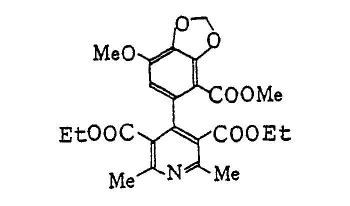

Diethyl 4-(methoxycarbonyl-3,4-methlenedioxy-5-methoxy)phenyl-2,6-dimethylpyridine-3,5-dicarboxylate Starting material: Diethyl 4-(2-methoxycarbonyl-3,4-methylenedioxy-5-methoxy)phenyl-2,6-dimethyl-1,4-dihydropyridine-3,5-dicarboxylate

Melting point: 134 - 135 °C (ethyl aceate-n-hexane)

Elemental anslysis (as C₂₃ H₂₅ NO₉)

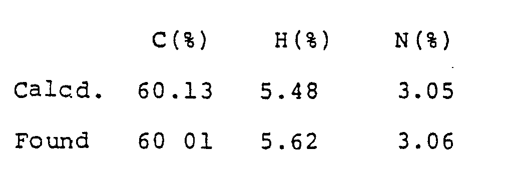

### Example 33

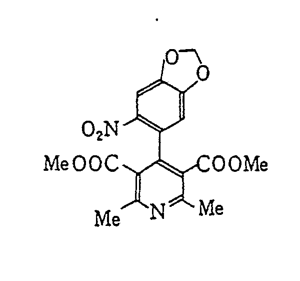

Dimethyl 4-(3,4-methylenedioxy-6-nitro)phenyl-2,6-dimethylpyridine-3,5-dicarboxylate Melting point: 163 - 164 °C (ethyl acetate)

Elemental analysis (as C₁₈ H₁₆ N₂ O₈)

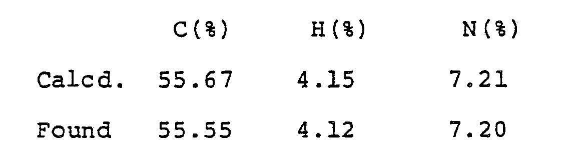

Starting material: Dimethyl 4-(3,4-methylenedioxy-6-nitro)phenyl-2,6-dimethyl-1,4-dihydropryidine-3,5-dicarboxylate

### Example 34

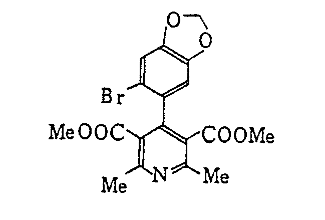

Dimethyl 4-(3,4-methylenedioxy-6-bromo)phenyl-2,6-dimethylpyridine-3,5-dicarboxylate Melting point: 107 - 108 °C

Elemental analysis (as C,ₛ H₁₆ NO₆Br)

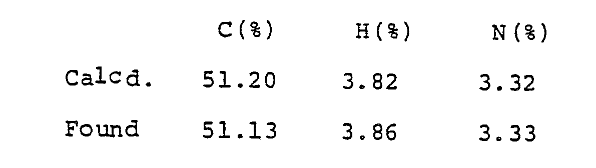

Starting material: Dimethyl 4-(3,4-methylenedioxy-6-bromo)phenyl-2,6-dimethyl-1,4-dihydropyridine-3,5-dicarboxylate

### Example 35

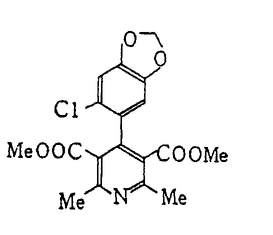

Dimethyl 4-(3,4-methylenedioxy-6-chloro)phenyl-2,6-dimethylpyridine-3,5-dicarboxylate Melting point: 104 - 105 °C

Elemental analysis (as C₁₈ H₁₆ NO₆ Cl)

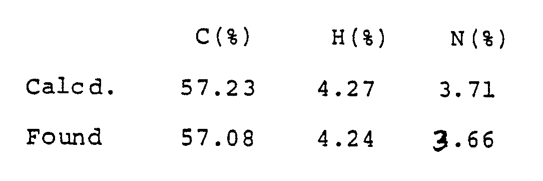

Starting material: Dimethyl 4-(3,4-methylenedioxy-6-chloro)phenyl-2,6-dimethyl-1,4-dihydropyridine-3,5-dicarboxylate

### Example 36

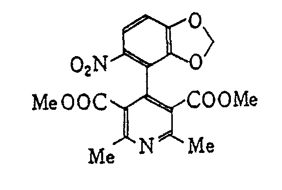

Dimethyl 4-(2,3-methylenedioxy-6-nitro)phenyl-2,6-dimethylpyridine-3,5-dicarboxylate Melting point: 118 - 120 °C (ethyl acetate-n-hexane)

Elemental analysis (as C₁₈ N₁₆ N₂O₈)

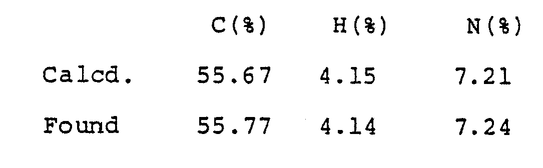

Starting material: Dimethyl 4-(2,3-methylenedioxy-6-nitro)phenyl-2,6-dimethyl-1,4-dihydropyridine-3,5-dicarboxylate

### Example 37

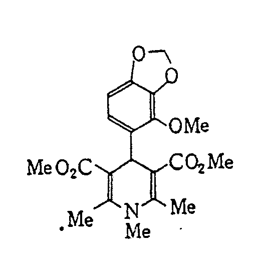

A suspension of 0.45 g of 60 % sodium hydride in 15 ml of anhydrous tetrahydrofuran was maintained at 5-10 °C, and to the suspension was added dropwise a solution of 3.50 g of dimethyl 2,6-dimethyl-4-(2-methoxy-3,4-methylenedioxy)phenyl-1,4-dihydropyridine-3,5-dicarboxylate in 35 ml of dry tetrahydrofuran. The mixture was stirred at room temperature for 1 hour, and then at 30 °C for 30 minutes, and after adding thereto 1.99 g of iodomethane, the mixture was stirred at room temperature for 12 hours. After adding thereto water, the mixture was extracted with chloroform (100, 50 ml). The organic layer was washed with water and saturated brine successively, and dried over magnesium sulfate (anhydrous), Magnesium sulfate was removed by filtration, and the solvent removed by distillation. The obtained crude crystals were subjected to column chromatography and eluted by n-hexaneethyl acetate (3 :1) to give dimethyl 2.6-dimethyl-4-(2-methoxy-3,4-methylenedioxy)phenyl-1-methyl)1,4-dihydropyridine-3,5-dicarboxylate.

Melting point: 152 - 154 °C (ethyl acetate-n-hexane)

Elemental analysis (as C₂₀ H₂₃ NO₇)

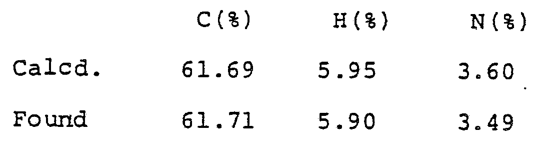

In similar manner to Example 37 the following compounds were obtained.

### Example 38

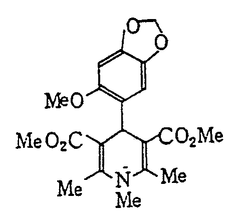

Dimethyl 2,6-dimethyl-4-(2-methoxy-4,5-methylenedioxy)phenyl-1-methyl-1,4-dihydropyridine-3,5-carboxylate

Melting point: 210 - 215 °C (ethyl acetate)

Elemental analysis (as C₂₀ H, NO₇)

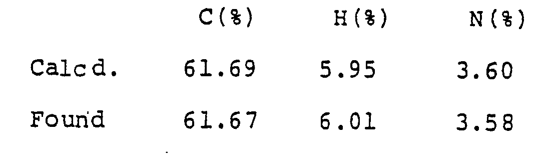

Starting material: Dimethyl 2,6-dimethyl-4-(2-methoxy-4,5-methylenedioxy)phenyl-1,4-dihydropyridine-3,5-dicarboxylate

### Example 39

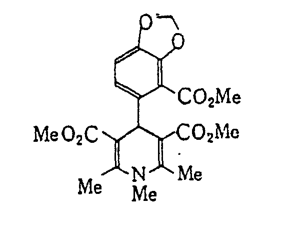

Dimethyl 2,6-dimethyl-4-(2-methoxycarbonyl-3,4-methylenedioxy)phenyl-1-methyl-1,4-dihydropyridine-3,5-dicarboxylate

Melting point: 137 - 139 °C (ethyl acetate-n-hexane)

Elemental analysis (as C2, H₂₃ NOs)

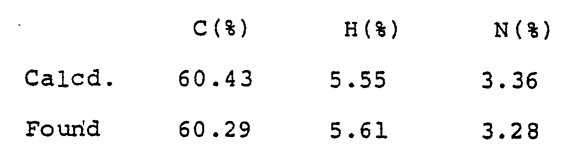

Starting material: Dimethyl 2,6-dimethyl-4-(2-methoxycarbonyl-3,4-methylenedioxy)phenyl-1,4-dihydropyridine-3.5-dicarboxylate

### Example 40

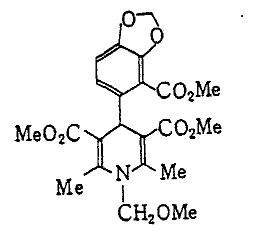

To a suspension of 0.30 g of 60 % sodium hydride in 7.5 ml of dry dimethyl formamide, was added a solution of 2.50 g of dimethyl 2,6-dimethyl-4-(2-methoxycarbonyl-3,4-methylenedioxy)phenyl-1,4-dihydropyridine-3,5-dicarboxylate in 15 ml of dimethylformamide. The mixture was stirred at room temperature for 30 minutes, and after adding thereto'0.09 g of sodium iodide and 0.75 g of methoxymethyl chloride, the mixture was stirred for 1.5 hour. After adding thereto water, the mixture was extracted with ether (50 ml x 2, 20 ml). The organic layer was washed with saturated aqueous sodium hydrogen carbonate and saturated brine successively, and dried over anhydrous magnesium sulfate. Magnesium sulfate was ' removed by filtration, and after removing the solvent by distillation, the obtained crude crystal were subjected to column chromatography (silica gel: 85 g) and eluted by n-hexane-ether (2 :3) - (1 :2) to give 0.72 g of dimethyl 2,6-dimethyl-4-(2-methoxycarbonyl-3,4-methylenedioxy)phenyl-1-methoxymethyl-1,4-dihydropyridine-3,5-dicarboxylate:
Melting point: 156 - 158 °C
Elemental analysis (as C22 H₂₅ NO,)

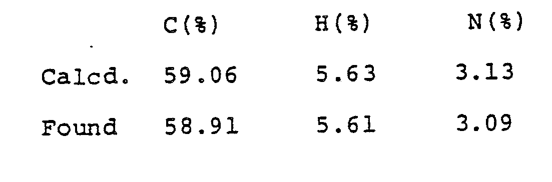

### Example 41

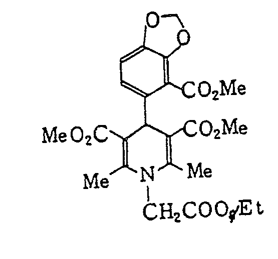

To a suspension of 0.74 g of 60% sodium hydride in 10 ml of dry methyl formamide, was added a solution of 5.0 g of dimethyl 2,6-dimethyl-4-(2-methoxycarbonyl-3,4-methylenedioxy)phenyl-1,4-dihydropyridine-3,5-dicarboxylate in 25 ml of dimethylformamide. The mixture was stirred at room temperature for 30 minutes, and after adding thereto 1.86 g of sodium iodide and 4.14 g of ethyl bromoacetate, the mixture was stirred for 1.5 hour. The solvent was removed by distillation, and after adding thereto water, the mixture was extracted with ethyl acetate. The organic layer was washed with water and saturated brine successively, and dried over anhydrous magnesium sulfate. Magnesium sulfate was removed by filtration, and after removing the solvent by distillation, the obtained crude crystals were subjected to column chromatography (silica gel: 190 g) and eluted by n-hexane-ether (2 :3) to give 0.65 g of dimethyl 1- ethoxycarbonylmethyl-2,6-dimethyl-4-(2-methoxycarbonyl-3,4-methylenedioxy)phenyl-1,4-dihydropyridine-3,5-dicarboxylate

Melting point: 132 - 133 °C (ethyl acetate-n-hexane)

Elemental analysis (as C₂₄ H₂₇ NO₁₀)

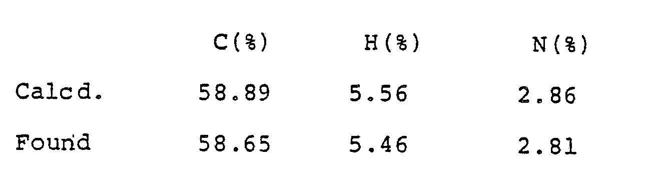

### Example 42

10 g of 2-methoxycarbonyl-3,4-methylenedioxybenzaldehyde and 11.9 g of 2-(N-benzyi-N-methylamino)ethylacetoacetate were dissolved in 84 ml of benzene and after adding thereto 0.5 ml of piperidine and 1.5 ml of acetic acid, the mixture was heated to reflux for 2.5 hours to be subjected to azeotropic dehydration. After cooling, benzene was added to the reaction mixture, and the mixture was washed with water, saturated sodium hydrogen carbonate solution and saturated brine successively, and dried over magnesium sulfate (anhyrous). Benzene was removed by distillation under reduced pressure, and the residue was dissolved in 200 ml of iso-propanol. After adding to the obtained solution 5.53 g of methyl 3-aminocrotonate, the mixture was heated to reflux for 36 hours. After cooling, iso-propanol was removed by distillation under reduced pressure, and the residue was subjected to silica gel column- chromatography (eluting solution: ethyl acetate-hexane mixture) to give yellow oily material of 2-(N-benzyl-N-methylamino)ethyl methyl 4-(2-methoxycarbonyl-3,4-methylenedioxy)phenyl-2,6-dimethyl-1,4-dihydropryidine-3.5-dicarboxylate. This oily material was dissolved in HCI-saturated ethyl alcohol and after removing ethanol by distillation under reduced pressure and then treating with 100 ml of ether, crystals of the product (HCI salt)were obtained (11 g).

Melting point: 240 - 242 °C (70 % ethanol)

Elemental analysis (as C₂₉ H_{"} N₂O₈Cl)

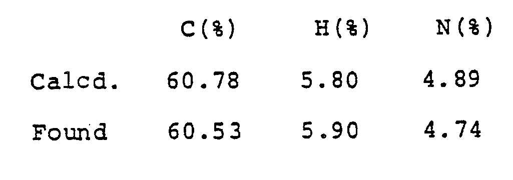

In similar manner to Example 42 the following, compounds were obtained.

### Example 43

2-Dimethylaminoethyl methyl 4-(2-methoxycarbonyl-3,4-methylenedioxy)phenyl-2,6-dimethyl-1,4-dihydropyridine-3.5-dicarboxylate

Melting point: 103 - 106 °C (methylene chloride-hexane)

Elemental analysis (as C₂₃ H₂₈ N₂O₈ 1/2 H₂O)

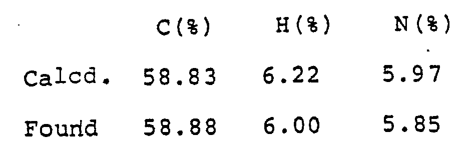

### Example 44

3-Dimethylaminopropyl methyl 4-(2-methoxycarbonyl-3,4-methylenedioxy)phenyl-2,8-dimethyl-1,4-dihydropyridine-3,5-dicarboxylate (oily material)
Mass spectrum (m/z): 474 (M⁺)
Nuclear magnetic resonance Spectrum:
   8: 2.00 (2H, m, -CH₂-CH₂-CH₂-), 2.18, 2.28 (each 3H, s, 2,6-Me), 2.70 (3H, s, -N(Me)₂), 2.90 (2H, broad, -CH₂N), 3.48 (3H, s. -COOMe), 3,85 (3H, s. -COOMe), 4.00 (2H, t, -OCH₂-), 5.30 (1 H, s, ≡C-H), 6.04 (2H, s, -OCH₂O-), 6.76 (1 H, d, Aromatic H), 6.96 (1 H, d, Aromatic H), 8.94 (1 H, s, -NH)

### Example 45

2-Pyrolidinoethyl methyl 4-(2-methoxycarbonyl-3,4-methylenedioxy)phenyl-2,6-dimethyl-1,4-dihydropyridine-3,5-dicarboxylate (oily material)

Mass spectrum (m/z): 486 (M⁺)

Nuclear magnetic resonance (DMSO-d₆, TMS)

8:1.62 (4H, m, 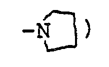 , 2,20, 2,24 (each 3H, s, 2,6-Me), 2.00 - 2.70 (6H, m, -CH₂N x 3), 3.50 (3H, s, -COOMe). 3.84 (3H, s, -COOMe), 4.04 (2H, t, -OCH₂-), 5,30 (1 H, s, C-H), 6.02 (2H, d, -OCH₂O-), 6.75 (1 H, d, Aromatic H), 6.94 (1 H, d, Aromatic H) 8,76 (1 H, s, NH)

### Example 46

To a mixture of 60 ml of benzene and 60 ml of water, was added 4.1 ml of 60 % nitric acid, and then 6 g of (2-benzylmethylamino)ethyl methyl 4-(2-methoxycarbonyl-3,4-methylenedioxy)phenyl-2,6-dimethyl-1,4-dihydropyridine-3,5-dicarboxylate HCI sat was added to the obtained mixture. To the suspension thus obtained, was added 2.5 g of sodium nitrite in 3 portions, and the mixture thus obtained was stirred for 30 minutes at room temperature. While cooling, the mixture was alkalified with 10 % sodium hydroxyde solution, and extracted with chloroform. The extract was washed with water and saturated brine, successively, and dried over anhydrous magnesium sulfate. Chloroform was removed by distillation under reduced pressure, and the residue was subjected to column chromatography (eluting solution: ethyl acetate-hexane mixture) to give 4.2 g of colorless oily material of (2-benzylmethylamino)ethyl methyl 4-(2-methoxycarbonyl-3,4-methylenedioxy)phenyl-2,6-dimethylpyridine-3,5-dicarboxylate.

Mass spectrum:(m/z): 534 (M⁺), 503

Nuclear magnetic resonance (CDCl₃, TMS)

δ: 2.15 (3H, s, N-CH₃), 2.46 (2H, t, N-CH₂-) 2.48 (3H, s, 2,6-Me), 3.46 (2H, s, -CH₂-phenyl) 3.58 (3H, s, -COOMe), 3.64 (3H, s, -COOMe) 4.15 (2H, t, -O-CH₂-), 6.14 (2H, q, -OCH₂O-) 6.58 (1 H, d, Aromatic H), 6.86 (1 H, d, Aromatic H) 7.25 (5H, s, -Aromatic H)

In similar manner to Example 46 the following compounds were obtained.

### Example 47

2-Dimethylaminoethyl methyl 4-(2-methoxycarbonyl-3,4-methylenedioxy)phenyl-2,6-dimethylpyridine-3,5-dicarboxylate (oily material)

Mass spectrum (m/z): FAB 459 (M⁺+1)

Nuclear magnetic resonance (DMSO-d₆, TMS)

δ : 2.14 (6H, s, -NMe₂), 2.32 (2H, t, -CH₂N), 2.48, 2.52 (each 3H, s, 2,6-Me), 3.53 (3H, s, -COOMe) 3.60 (3H, s, -COOMe), 4.05 (2H, t, -OCH₂-) 6.21 (2H, d, -OCH₂O-), 6.54 (1 H, d, Aromatic H) 7.13 (1 H, d, Aromatic H)

### Example 48

3-Dimethylaminopropyl methyl 4-(2-methoxycarbonyl-3,4-methylenedioxy)phenyl-2,6-dimethylpyridine-3,5-dicarboxylate (oily material)

Mass spectrum (m/z): 472 (M⁺)

Nuclear magnetic resonance (DMSO-d₆, TMS)

δ : 1.48 (2H, m, -CH₂CH₂CH₂-), 2,14 (6H, s, -NMe₂) 2.14 (2H, t, -CH2N), 2.50, 2.52 (each 3H, s, 2,6-Me), 3.52 (3H, s, -COOMe), 3.58 (3H, s, -COOMe). 3.98 (2H, t, -OCH₂-), 6.20 (2H, d, -OCH₂0-), 6.55 (1H, d, Aromatic H), 7.14 (1 H, d, Aromatic H)

### Example 49

2-Pyrolidinoethyl methyl 4-(2-methoxycarbonyl-3,4-methylenedioxy)phenyl-2,6-dimethylpyridine-3.5-dicarboxylate (oily material)

Mass spectrum (m/z): FAB 485 (M⁺ + ₁)

Nuclear magnetic resonance (DMSO-d₆, TMS)

δ : 1.70 (4H, m, 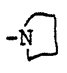 ), 2.50, 2.52 (each 3H, s, 2,6-Me), 3.52 (3H, s, -COOMe), 3.58 (3H, s, -COOMe), 4.10 (2H, t. -OCH₂-), 6.22 (2H, d, -OCH₂O-), 6.55 (1 H, d, Aromatic H), 7.15 (1 H, d, Aromatic H)

### Example 50

2.38 g of 5-methoxy-2-methoxycarbonyl-3,4-methylenedioxybenzaldehyde and 2.74 g of 2-(N-benzyl-N-methylamino)ethylacetoacetate was dissolved in 50 ml of benzene, and after adding thereto 0.2 ml of piperidine and 0.6 mlof acetic acid, the mixture was heated to reflux for 2 hours. After cooling, 50 ml of benzene was added, and the mixture was washed with water, saturated sodiumhydrogen carbonate solution and saturated NaCl solution, successively and dried over anhydrous magnesium sulfate. Benzene was removed by distillation under reduced pressure, and the residue was dissolved in 50 ml of iso-propanol. 1.27 g of methyl 3-aminocrotonaate was added to the mixture, and the mixture was heated to reflux for 24 hours. After cooling, iso-propanol was removed by distillation under reduced pressure, and the residue was subjected to column chromatography (silica gel) (eluting solution: ethyl acetate-hexane mixture) to give 2-(N-benzyl-N-methylamino)ethyl methyl 4-(5-methoxy-2-methoxycarbonyl-3,4-methylenedioxy)phenyl-2,6-dimethyl-1,4-dihydropyridine-3,5-dicarboxylate as oily material. This oily material was treated with ethanolic HCI, and crystallized from acetone to give 1.88 g of the HCI salt of the product.

Melting point: 215 - 217 °C (acetone)

Elemental analysis (as C₃₀ H₃₅ N₂ O₉ CI)

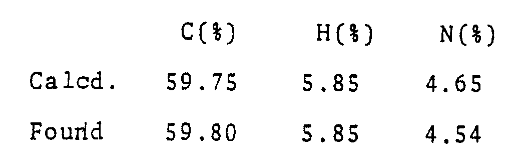

In similar manner to Example 50 the following compounds were obtained.

### Example 51

3-Dimethylaminopropyl methyl 4-(5-methoxy-2-methoxycarbonyl-3,4-methylenedioxy)phenyl-2,6-dimethyl-1,4-dihydropyridine-3,5-dicarboxylate HCI salt

Melting point: 137-139 °C

Elemental analisis (as C₂₅ H₂₃ N2 O₉ Cl)

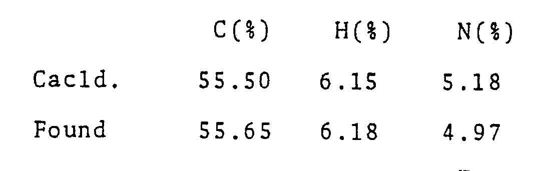

### Example 52

2-Dimethylaminoethyl methyl 4-(5-methoxy-2-methoxycarbonyl-3,4-methylenedioxy)phenyl-2,6-dimethyl-1,4-dihydropyridine-3,5-dicarboxylate HCI salt

Melting point: 119 - 121 ° C (acetone)

Elemental analysis (as C₂₄ H₃₁ N₂ O₉ Cl)

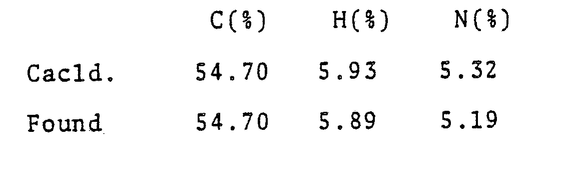

### Example 53

1.02 g of (2-(N-benzyl-N-methylamino)ethyl methyl 4-(5-methoxy-2-methoxycarbonyl-3,4-methylenedioxy)phenyl-2,6-dimethyl-1,4-dihydropyridine-3,5-dicarboxylate was suspended in a mixture of 10 ml of water, 10 ml of dioxane and 0.64 ml of 61 % nitric acid, and after adding thereto 280 mg of sodium nitrite in 2 portions, the mixture was stirred for 30 minutes at room temperature. The mixture was alkalified with 1 N-sodium hydroxide solution, and extracted with ethyl acetate. The extract were washed with water and saturated NaCl solution successively, and dried over anhydrous magnesium sulfate. The solvent was removed by distillation under reduced pressure, and the residue was subjected to silica gel column chromatography to give 850 mg of yellow material of 2-(N-benzyl-N-methylamino)ethyl methyl 4-(5-methoxy-2-methoxycarbonyl-3,4-methylenedioxy)phenyl-2,6-dimethylpyridine-3,5-dicarboxylate.

Mass spectrum (m/z): 564(M⁺), 533

Nuclear magnetic resonance (CDCl₃, TMS)

δ: 2.16 (3H, s, N-Me), 2.48 (2H, t, N-CH,-), 2.60, 2.63 (each 3H, s, 2,6-Me), 3.47 (3H, s, -CH₂-phenyl), 3.60 (6H, s, -COOMe) 3.84 (3H, s, -OMe), 4.14 (2H, t, -OCH₂-), 6.10 (2H, d, d, -OCH₂O-), 6.32 (1H, s, Aromatic

H), 7.25 (5H, s, Aromatic H)

In similar manner to Example 53 the following compounds were obtained.

### Example 54

3-Dimethylaminopropyl methyl 4-(5-methoxy-2-methoxycarbonyl-3,4-methylenedioxy)phenyl-2,6-dimethylpyridine-3,5-dicarboxylate

Melting point: 104 -106 °C (ethyl acetate-hexane)

Elemental analysis (as C25 H₃₀ N₂O₉)

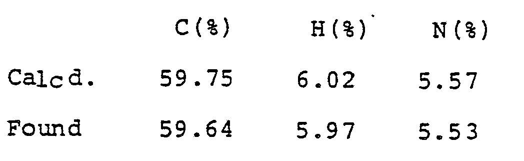

### Example 55

2-Dimethylaminoethyl methyl 4-(5-methoxy-2-methoxycarbonyl-3,4-methylenedioxy)phenyl-2,6-dimethylpyridine-3,5-dicarboxylate

Melting point: 78 -80 °C (ethyl acetate-hexane)

The compounds of Examples 42 - 55 are represented by the following formula:

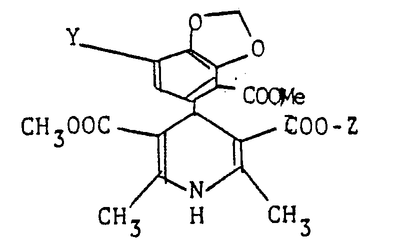

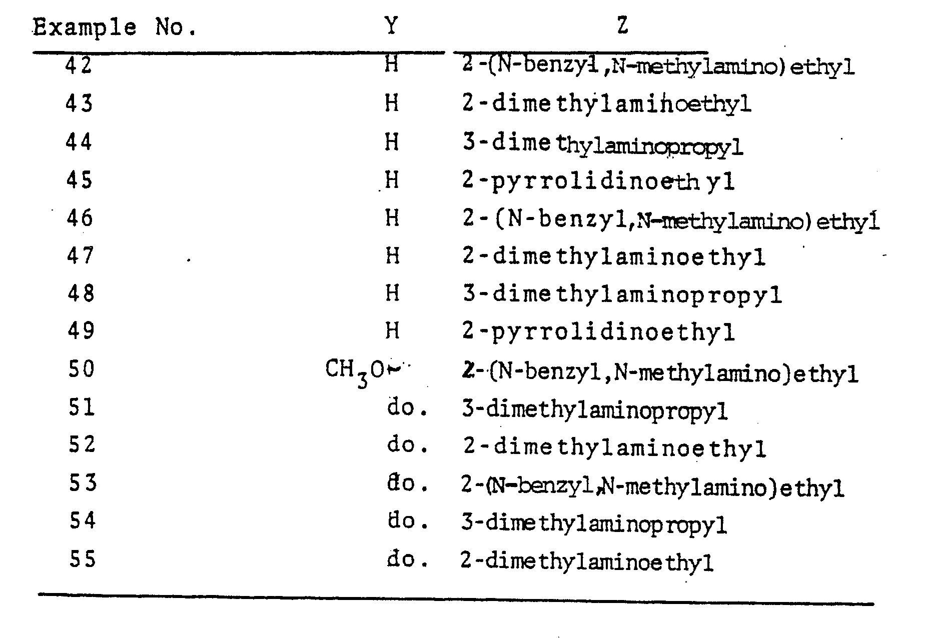

Some other compounds of formula (I) and (I-B) are shown below in Examples A to F and M to V. Compounds usable as intermediate in the production of pyridine derivatives described herein are shown below in Examples G to L.

### Example A

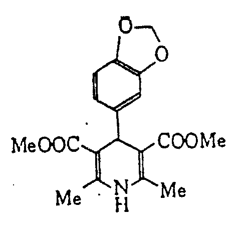

3.0 g of piperonal and 2.32 g of methyl acetoacetate was dissolved in 30 ml of benzene, and after adding thereto 0.3 ml of acetic acid and 0.1 ml of piperidine, the mixture was heated to reflux for 1.5 hour to be subjected to azeotropic dehydration. After cooling, 30 ml of benzene was added to the mixture, and the mixture was washed with water, saturated sodium hydrogen carbonate solution and saturated NaCI solution, successively,and was dried over anhyrous magnesium sulfate. The solvent was removed by distillation, and the yellow material thus obtained was dissolved in 30 ml of iso-propanol; after adding to the solution 2.3 g of methyl 3-aminocrotonate, the mixture was heated to reflux for 17 hours. Iso-propanol was removed by distillation, and the residue was subjected to recrystallization in ethyl acetate to give 3.2 g of dimethyl 4-(3,4-methylenedioxy)phenyl-2,6-dimethyl-1,4-dihydropyridine-3,5-dicarboxylate.

Melting point: 202 - 203 °C

Elemental analysis (as C₁₈ H,, NO₆)

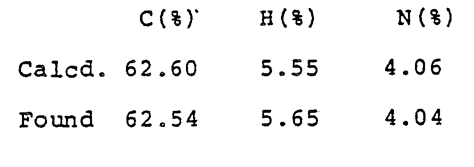

### Example B

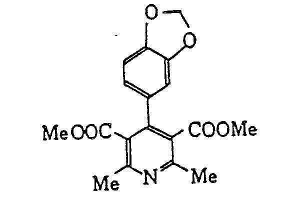

The diihydropyridine compound obtained by Example A (2 g) was dissolved in 76.5 ml of water and 9.6 ml of nitric acid (d=1.38), and the mixture was stirred for 15 minutes at 70°C. After cooling, the reaction mixture was alkalified with 10 % sodium hydroxide solution, and extraced with chloroform (70 ml x 2). The chloroform layer was washed with saturated NaCl solution, and dried over anhydrous sodium sulfate. Chloroform was removed by distillation and the residue was recrystallized from ethyl acetate-n-hexane to give 820 mg of dimethyl 4-(3,4-methylenedioxy)phenyl-2,6-dimethylpyridine-3,5-dicarboxylate.

Melting point: 121 - 122 °C

Elemental analysis (as C₁₈ H₁₇ NO₆)

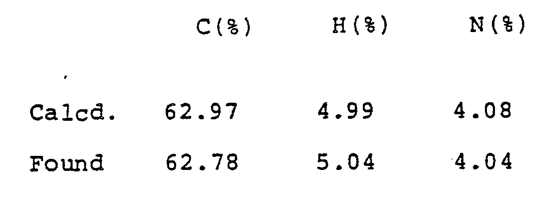

### Example C

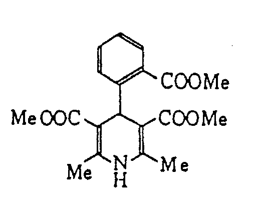

4.38 g of 2-methoxycarbonyl-benzaldehyde and 3.98 g of methyl acetoacetate was dissolved in 50 ml of benzene, and after adding thereto 0.3 ml of acetic acid and 0.1 ml of piperidine, the mixture was heated to reflux for 2 hours for azetropic dehydration. After cooling, 50 ml of benzene was added to the mixture, the mixture was washed with water, saturated sodium hydroxide solution and saturated NaCI solution, successively, and dried over anhydrous magnesium sulfate. The solvent was removed by distillation under reduced pressure, and the residue obtained was dissolved in 100 ml of iso-propanol, and after adding thereto 3.07 g of methyl 3-aminocrotonate, the mixture was heated to reflux for 19 hours. Iso-propanol was removed by distillation under reduced pressure, and the residue was recrystallized from ethyl acetate-n-hexane to give 5.32 g of dimethyl 4-(2-methoxycarbonyl)phenyl-2,6-dimethyl-1,4-dihydropyridine-3,5-dicarboxylate.

Melting point: 202 - 203 °C

Elemental analysis (as C₁₉ H₂₁ NO₆)

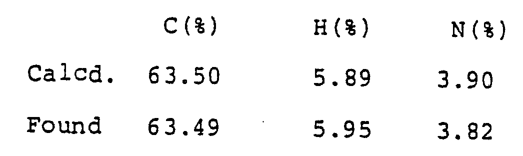

### Example D

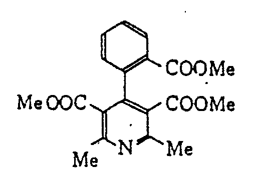

To 2.32 g of the dihydropyridine compound obtained by Example C, was added 43 ml of dioxane, 85 ml of water and 21.4 ml of nitric acid (d=1.38), and the mixture was stirred for 3 hours at 80 °C. After cooling, the mixture was alkalified with 10 % sodium hydroxide solution, and extracted with chloroform (70 ml x 2). The chloroform layer was washed with saturated NaCI solution, and dried over anhydrous magnesium sulfate. Chloroform was removed by distillation under reduced pressure, and the residue was recrystallized from ethyl acetate-n-hexane to give 700 mg of crystal of dimethyl 4-(2-methoxycarbonyl) phenyl-2,6-dimethylpyridine-3,5-dicarboxylate.

Melting point: 80 -81 °C

Elemental analysis (as C,9 H,9 NO₆)

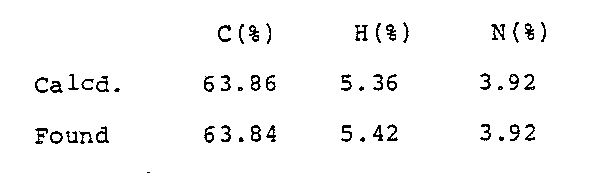

### Example E

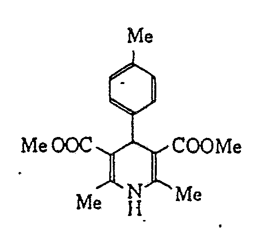

3.6 g of 4-methylbenzaledhyde and 3.5 g of acetoacetic acid ethyl ester was dissolved in 30 ml of benzene, and after adding thereto 0.6 ml of acetic acid and 0.2 ml of piperidine, the mixture was heated to reflux for 1.5 hours for azeotropic dehyration. After cooling, 30 ml of benzene was added to the mixture, and the mixture was washed with water, saturated sodium hydrogen carbonate and saturated NaCI solutions, successively, and dried over anhydrous magnesium sulfate. Benzene was removed by distillation, and the residue was dissolved in 60 ml of iso-propanol, and after adding thereto 3.5 g of methyl 3-amino crotonate, the mixture was heated to reflux overnight. lso-propanol was removed by distillation, and the residue was recrystallized from ethyl acetate to give 6.85 g of crystal of dimethyl 4-(4-methyl)phenyl-2,6-dimethyl-1,4-dihydropyridine-3,5-dicarboxylate.

Melting point: 175 - 176 °C

Elemental analysis

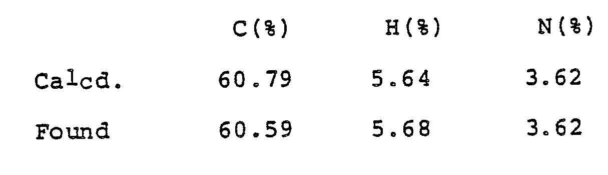

### Example F

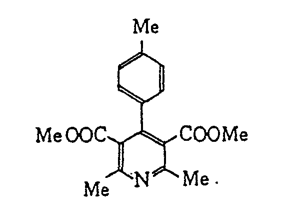

3.15 g of dihydropyridine compound obtained by Example E was dissolved in 45 ml of dioxane and 45 ml of water, and after adding gradually 22.5 ml of nitric acid (d = 1.38) while warming at 80 °C, the mixture was stirred for 15 minutes. After cooling, the mixture was alkalified with 10 % sodium hydroxide solution, and was extracted with chloroform (100 ml x 2). The extract was washed with water and saturated sodium chloride solution, successively. Chloroform was removed by distillation under reduced pressure and the residue was recrystallized from ethyl acetate -n-hexane to give .23 g of dimethyl 4-(4-methyl)phenyl-2,6-dimethylpyridine-3,5-dicarboxylate.

Melting point: 84 - 85 °C

Elemental analysis (as C₁₈ H,₉ NO₄)

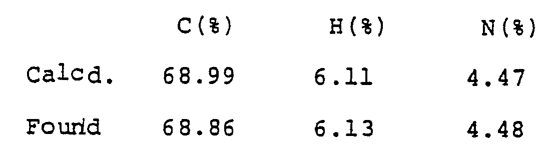

### Example G

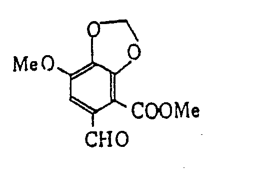

6.17 g of 3,4-methylenedioxy-5-methoxybenzaldehydecyclohexylimine was dissolved in 200 ml of dry tetrahydrofuran, the mixture was cooled to -78 °C in argon atmosphere, and after adding thereto 15 % n-butyl lithium-n-hexane solution (15.9 ml) dropwise gradually, the mixture was stirred for 30 minutes. After adding thereto dry ice powder (2 g), the mixture was stirrred for 15 minutes, and the temperature of the solution returned to room temperature. After adding to the mixture 150 ml of ether and 175 ml of 15% HCI, the mixture was stirred vigorously overnight. The ether layer was separated, and dissolved in 200 ml of 10 % potassium hydroxide solution. After cooling with ice, the mixture was acidified with concentrated hydrochloric acid, and extracted with ethyl acetate (150 ml x 2). The mixture was washed with saturated NaCI solution, and dried over anhydrous magnesium sulfate. The solvent was removed by distillation under reduced pressure, and yellow crystals obtained were washed with ether to give 1.15 g of 2-formyl-4-methoxy-5,6-methylenedioxybenzoic acid.

Molecular weight: 224.17 (C₁₀ H₈O₆)

Mass 224 (M⁺), 196, 148, 120

The above obtained carboxylic acid (1.0 g) was suspended in 50 ml of ether, and after adding thereto diazomethane-ether solution while cooling gradually, until the production of nitrogen gas stopped, the mixture was treated with acetic acid to decompose excess diazomethane. After adding to the mixture 50 ml of ethyl acetate, the mixture was washed with saturated sodium hydrogen carbonate solution and saturated NaCI solution, and dried over anhydrous magnesium sulfate. Ether was removed by distillation under reduced pressure, and the residue was washed with ether to give 0.89 g of methyl 2-formyl-4-methoxy-5,6-methylenedioxybenzonate showing a melting point of 138 -139 °C.

Molecular weight: 238.20 (C₁₁ H₁₀ O₆)

N.M.R. (CDCl₃,500MHz)

3.96 (3H, s, -Me), 3.99 (3H, s, -COOMe). 6.18 (2H, s, -OCH₂O-), 7.26 (1 H, s. ring H), 10.34 (1 H, s, -CHO)

The following compounds were also prepared in conventional manners. The quoted temperatures are the melting points of the compounds.

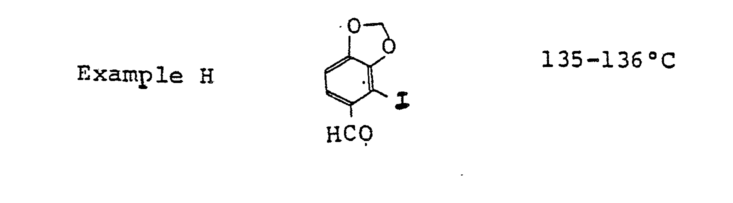

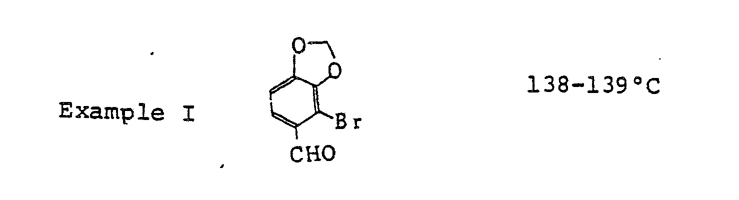

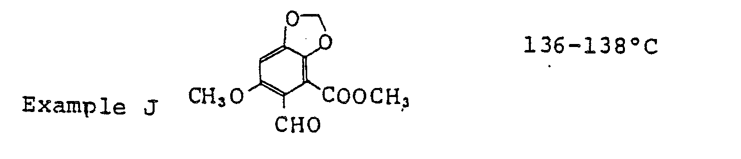

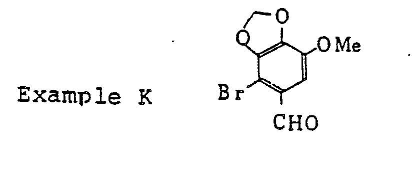

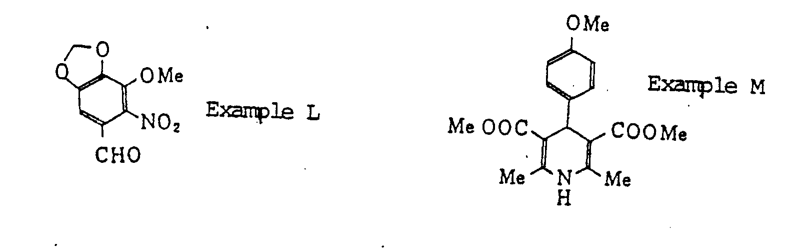

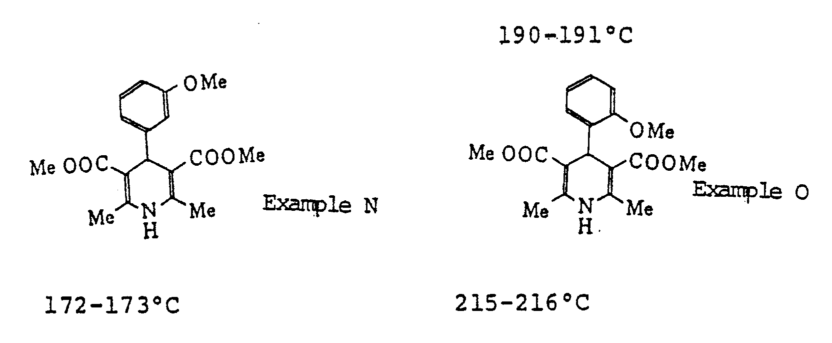

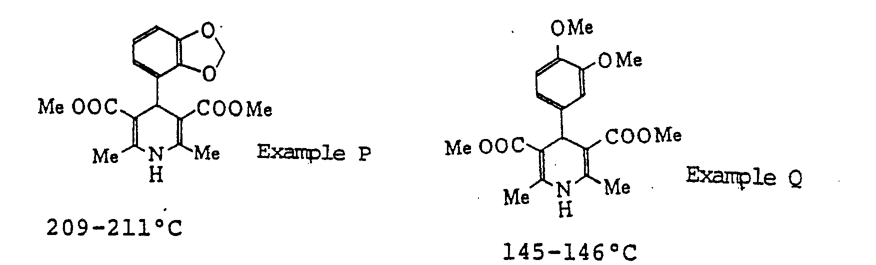

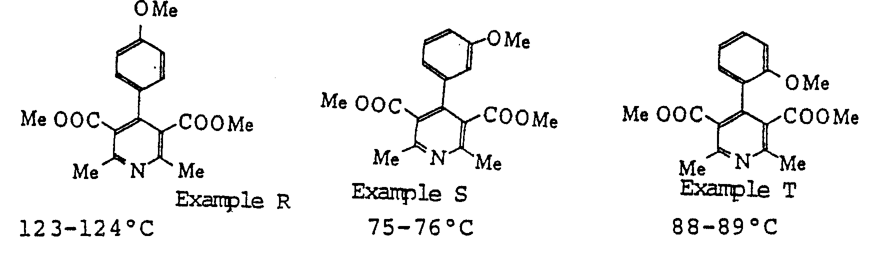

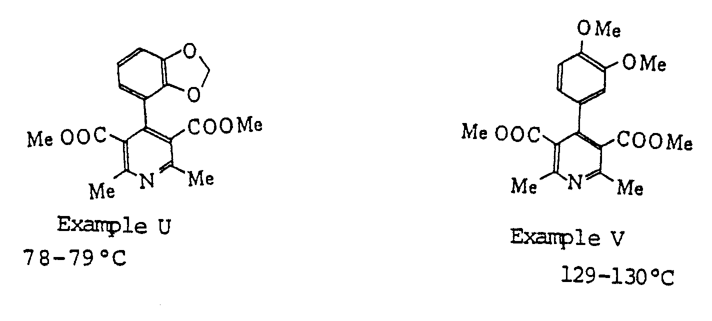

The Example P compound was prepared as follows:
4.5 g of 2,3-methylenedioxybenzaldehyde and 3.5 g of methyl acetoacetate were dissolved in 50 ml of benzene, and after adding thereto 0.2 ml of piperidine and 0.6 ml of acetic acid, the mixture was refluxed under heating for 2.5 hours azeotropic dehydration. After cooling, 50 ml of benzene was added to the mixture, and the mixture was washed with water, saturated aqueous NaHCO, and saturated NaCI solution, successively. This mixture was dried, and the solvent was removed by distillation under reduced pressure. To the residue were added 3.5 g of 3-aminocrotonic acid methyl ester and 50 ml of iso-propanol, and the mixture was refluxed under heating overnight. After cooling, iso-propanol was removed by distillation under reduced pressure, and the residue was recrystallized from ether to give 7.6 g of crude crystal of dimethyl 4-(2,3-methylenedioxy)phenyl-2,6-dimethyl-1,4-dihydropyridine-3,5-dicarboxylate.

The Example U compound was prepared as follows:
4.27 g of dimethyl 4-(2,3-methylenedioxy)phenyl-2,3-dimethyl-1,4-dihydropyridine-3,5-carboxylate was suspended in a mixture of 30 ml of dioxane and 30 ml of water, and after adding thereto 9.2 ml of conc. HNO₃ and 1.0g of NaNO₂, the mixture was stirred for 30 minutes at room temperature. The mixture was alkalified with 10%NaOH solution, and extracted with chloroform. The extract was washed with water and sat.NaCL solution, and dried. The solvent was removed by distillation under reduced pressure, and the residue was collected by filtration and recrystallized from ethyl acetate-n-hexane to give 2.6 g of crystal of dimethyl 4-(2,3-methylenedioxy)phenyl-2,6-dimethylpyridine-3,5-dicarboxylate.

## Claims

1. A pyridine derivative of the following formula (I), or a salt thereof: 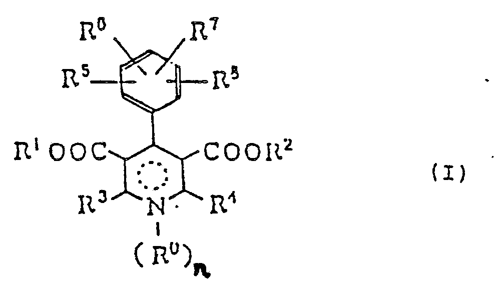 wherein R¹ and R² are the same or different and selected from C, -C6 alkyl groups and groups represented by 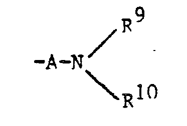 (wherein A is a C₁-C₆ alkylene; and R⁹ is C₁-C₆ alkyl and R¹⁰ is C₁-C₆ alkyl or aralkyl or R⁹ and R¹⁰ together form a 5-or 6-membered ring;
R³ and R⁴ are the same or different and selected from C₁-C₅ alkyl groups;
R⁵ and R⁶ are not both hydrogen but otherwise are the same or different and selected from hydrogen and halogen atoms and C₁-C₆ alkoxy, (C, -C₆ alkoxy)carbonyl and nitro groups;
R' and R⁸ together form C, -C₆ alkylenedioxy (that is, 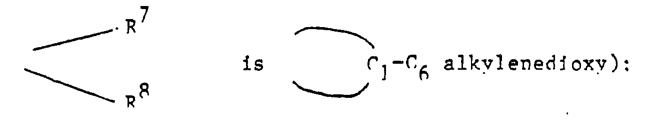 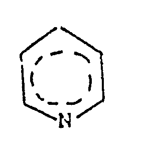 is fully unsaturated or has only a 2,3-position double bond and/or a 5,6-position double bond;
R° is a hydrogen atom, or a C₁-C₆ alkyl group which may be substituted by (C₁-C₆ alkoxy)carbonyl or C, - C₆ alkoxy; and n is 0 or 1 according to the structure of the attached heterocyclic ring.

2. A compound according to claim 1 wherein R⁵ and R⁶ are different and selected from hydrogen and halogen atoms and C₁-C₆ alkoxy, (C₁-C₆ alkoxy)carbonyl and nitro groups.

3. A compound according to claim 1 or 2 wherein R¹⁰ is C, -C₆ alkyl or aralkyl, or R⁹ and R¹⁰ together form a 5-or 6-membered ring.

4. A compound according to claim 1 which is dimethyl 4-(2-methoxycarbonyl-3,4-methylenedioxy)-phenyl-2,6-dimethyl-1-4-dihydropyridine-3,5-dicarboxylate or a salt thereof, or dimethyl 4-(2-methoxycarbonyl-3,4-methylen6dioxy-5-methoxy)-2,6-dimethylpyridine-3,5-dicarboxylate or a salt thereof.

5. A pyridine derivative of the following formula (I-B) or a salt thereof : 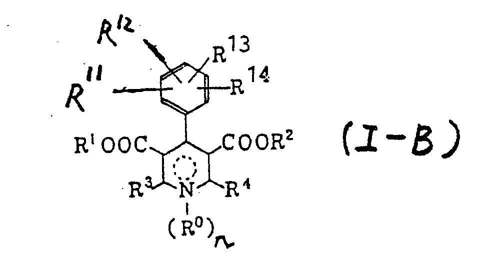 wherein R¹, R², R³, R⁴ are as defined in claim 1 above, R¹³ and R¹⁴ are the same or different and are selected from hydrogen, halogen, C₁-C₆ alkoxy, (C₁-C₆ alkoxy)carbonyl and nitro; R¹¹ and R¹² are both hydrogen atoms, or together form C₁-C₆ alkylenedioxy (with the proviso that when R¹¹ and R¹² are both hydrogens, R¹³ and R¹⁴ are not both hydrogens); and the remaining symbols are as defined in claim 1.

6. A pharmaceutical composition containing a compound according to any preceding claim.

7. The use of a compound as defined in claim 1, or as defined in claim 5, in the preparation of a medicament for the treatment or prevention of liver damage.

8. A method of preparing a compound as defined in claim 1, or as defined in claim 5, the method being substantially as described herein e.g. as described in any Example.

9. A compound which is usable as an intermediate for the production of a pyridine derivative as defined in claim 1, or as defined in claim 5, which compound is selected from any one of Example G, H, I, J, K and L herein.
